# EUROPEAN PATENT APPLICATION

(11) **EP 3 421 593 A1**
(43) Date of publication of application: **02.01.2019**
(21) Application number: 17305841.3
(22) Date of filing: 30.06.2017
(51) Int. Cl.: C12N 5/16

(54) **METHOD FOR OBTAINING HAPLOID CELLS**

(71) Applicant: Centre National de la Recherche Scientifique, 75016 Paris (FR)
(72) Inventor: MECHALI, Marcel, 34980 Montferrier sur Lez (FR); GANIER, Olivier, 68730 Blotzheim (FR)
(74) Representative: Regimbeau

(57) **Abstract**

The invention relates to the use of an inhibitor of the activity or stability of the pre-replication complex, also called pre-RC, for the *in vitro* preparation of haploid eukaryotic somatic cells from eukaryotic somatic diploid cells, said inhibitor completely inhibiting the activity or stability of said pre-RC.

## Description

The invention relates to a method for obtaining haploid cells, in particular for obtaining haploid cells from somatic cells.

Cells are classified according to the ploidy level of their DNA content: haploid, etc.

Some animals such as yeasts or males of social insects are haploid, i.e. they carry a single set of chromosomes, while haploidy in mammals is exclusively restricted to mature germ cells.

A single copy of the genome provides the basis for genetic analyses where any recessive mutation of essential genes will show a clear phenotype due to the absence of a second gene copy. Most prominently, haploidy in yeast has been utilized for recessive genetic screens that have markedly contributed to the inventors' understanding of development, basic physiology, and disease.

Somatic mammalian cells carry two copies of chromosomes (diploidy) that obscure genetic analysis.

Near haploid human leukemic cells however have been developed as a high throughput screening tool. Although deemed impossible, some researchers have generated mammalian haploid embryonic stem cells from parthenogenetic mouse embryos. Haploid stem cells open the possibility of combining the power of a haploid genome with pluripotency of embryonic stem cells to uncover fundamental biological processes in defined cell types at a genomic scale. Haploid genetics has thus become a powerful alternative to RNAi or CRISPR based screens.

However, both near haploid human cells and mammalian haploid embryonic stem cells are extremely difficult to obtain. Such cells would be an invaluable tool in life sciences, both for genetic screening, but also as a new tool to modify the genome.

It would be also useful to obtain haploid cells in order to study some inherited pathologies, the genotype of which being autosomal dominant, in cells carrying the single-allele mutation. Another aspect could be the necessity to obtain tissue specific haploid cells in order to study the physiology of these cells by simplest genetics techniques.

The invention therefore intends to obviate these drawbacks and deficiencies.

One aim of the invention is to provide a method for producing haploid cells from somatic cells.

Another aim of the invention is to provide haploid somatic cells.

One other aim of the invention is to provide compounds or molecules that can stimulate the formation of haploid cells from differentiated non-germinal cells.

The present invention relates to the use of an inhibitor of the activity or stability of the pre-replication complex, also called pre-RC, for the preparation, preferably in vitro, of haploid eukaryotic somatic cells from eukaryotic somatic diploid cells, said inhibitor completely inhibiting the activity or stability of said pre-RC.

In the invention, Eukaryotic cells encompass all the eukaryotic cells such as metazoan cells, including plant cells, in particular Chlorobionta, yeast cells, insect cells, worm cells, frog cells, mammal cells ...

Advantageously, the invention relates to the use of an inhibitor of the activity or stability of the pre-replication complex, also called pre-RC, for the preparation, preferably in vitro, of haploid mammal somatic cells from mammal somatic diploid cells, said inhibitor completely inhibiting the activity or stability of said pre-RC.

The invention is based on the unexpected observation made by the inventors that a complete blockage of the DNA replication licensing, by destabilizing or inhibiting the activity of the pre-replication complex, does not kill cells but only inhibit their ability to duplicate DNA. These cells remain able to enter into mitosis and further complete cell division, providing two haploid sister cells from a mother diploid cell.

This observation is a breakthrough, since the prior art recited that blockage of DNA replication would only induce cell death or senescence, but it was absolutely unexpected that the cells can survive to this DNA replication blockage.

Within the context of the invention, "DNA replication blockage" means that cells are not able to carry out DNA replication (i.e. producing two identical replicas of DNA from one original DNA molecule), in particular by inhibiting the initiation of the DNA replication (i.e. by inhibiting the replication origin activation). Thus, in the invention, a DNA replication process which has begun but is blocked before the complete duplication of the parental DNA molecule is not considered as a "DNA replication blockage". The "DNA replication blockage" according to the invention in particular completely blocks initiation of DNA replication.

By "pre-replication complex", it is meant in the invention the protein complex that forms at the origin of replication during the initiation step of DNA replication. Formation of the pre-RC is required for DNA replication to occur. Accordingly, formation of the pre-RC is a very important part of the cell cycle.

Pre-RC complex comprises so far the following proteins: the Origin replication complex (ORC) 1-6 proteins, CDC6 protein, the chromatin licensing and DNA replication factor 1 (Cdt1) protein and minichromosome maintenance 2-7 proteins. Eukaryotic cells have the most complex pre-RC. After ORC1-6 bind the origin of replication, Cdc6 is recruited. Cdc6 recruits therefore the licensing factor Cdt1 and MCM2-7 complex. Cdt1 binding and ATP hydrolysis by the ORC and Cdc6 load MCM2-7 onto DNA.

It is known in the art that cyclin dependent kinases CDKs prevent formation of the replication complex during late G1, S, and G2 phases by excluding MCM2-7 and Cdt1 from the nucleus, targeting Cdc6 for degradation by the proteasome, and dissociating ORC1-6 from chromatin via phosphorylation.

Proteolytic regulation of Cdt1 is also a mean to block pre-RC activity. This proteolysis is shared by higher eukaryotes including *Caenorhabditis elegans, Drosophila melanogaster, Xenopus laevis,* and mammals. In metazoans, another mechanism to prevent re-replication; during S and G2, involve the geminin protein which binds to Cdt1 and inhibits Cdt1 from loading MCM2-7 onto the origin of replication. Thus advantageously, the invention relates to the use of an inhibitor as defined above, this inhibitor being, without limitation
- The geminin protein, the expression of which being enforced to inhibit the loading or MCM2-7 complex,
- An activator of the degradation of the Cdt1 protein, or a modified protein which is inducibly degraded (phosphodegron), or excluding the protein form the nucleus,

Said inhibitor, used in the invention completely affects DNA replication. It is particularly relevant to block DNA replication initiation in synchronized cells, in order to simultaneously block the replication in all the cells before the initiation of the replication. When cells are not synchronized, i.e. all the cells are in same step (G1, S, G2 or M phase) of the cell cycle, the cells which are in the G1 phase when the inhibitor is applied would be the one that could efficiently give rise to haploid cells further to the M phase.

If replication has started and then blocked, cells will activate the DNA damage checkpoint and be blocked at the G2/M transition. If the licensing of replication is partially inhibited, some DNA replication will fire but not enough: in this case, cells will enter into M phase with partially replicated chromatids that will induce mitotic catastrophes.

The senescence is induced by cells that did not replicate but do not manage to divide (only a fraction of unlicensed cells managed to divide).

Advantageously, the invention relates to the use as defined above, for the preparation, in particular *in vitro,* of haploid eukaryotic somatic cells from eukaryotic somatic diploid cells which are in the G1 phase of the cell cycle, said inhibitor completely inhibiting the activity or stability of said pre-RC.

Advantageously, the invention relates to the use as defined above, for the preparation, in particular *in vitro,* of haploid mammal somatic cells from mammal somatic diploid cells which are in the G1 phase of the cell cycle, said inhibitor completely inhibiting the activity or stability of said pre-RC.

The above use of an inhibitor of the activity or stability of the pre-RC complex allows to obtain, from somatic diploid cells which are particularly in the G1 phase of the cell cycle, haploid cells. These cells are called "haploid somatic cells" because they arbor all the features of a somatic differentiated cell, but the DNA content is reduced by half compared to the somatic diploid cells.

The "haploid somatic cells" according to the invention are completely different from the natural germinal haploid cells, and cannot directly, when fused to the nucleus of a spermatozoid or an oocyte, carry out the early steps of development, i.e. reproduce fecundation and embryo development.

Advantageously, the invention relates to the use as defined above, wherein said inhibitor is an inhibitor of the activity or expression of the chromatin licensing or of the CDT1 protein, or an inhibitor of one of the following proteins: ORC1-6. CDC6, MCM2-7 proteins or the usage of any inhibitor of these latter factors, like geminin, inhibitor of CDT1.

In one another advantageous embodiment, the invention relates to the use as defined above, wherein said inhibitor is a formulation of geminin protein, said formulation comprising an amount of geminin protein at least 1-fold higher compared to the cellular endogenous amount of geminin protein.

Advantageously, the invention relates to the use as defined above wherein said inhibitor is a formulation of geminin protein, said geminin protein being expressed during G1 phase of the cell cycle whereas the natural endogenous geminin is not expressed during G1 phase of the cell cycle.

Indeed, as mentioned above, expressing a geminin protein during G1 phase will inhibit PreRC formation and thus inhibit cell division.

Advantageously, the invention relates to the use as defined above, wherein said inhibitor is a non proteasome-degradable form of the Geminin protein.

The inventors demonstrated, as shown in the Example section, that a non-degradable mutant of the Geminin protein inhibits Cdt1 and induces a DNA replication blockage by inhibiting the Pre-RC complex.

Advantageously, the invention relates to the use as defined above, wherein said non-proteasome-degradable form of the Geminin protein lacks the following sequence: **RxTLKxzQx.** (SEQ ID NO : 2), wherein x represents any amino acid and z represent a leucine (L) or a valine (V).

The inventors show that advantageously, a mutated form of the Geminin protein, in which the destruction box domain is deleted, acts as an efficient inhibitor of DNA replication, and thus is useful to produce haploid somatic cells from somatic diploid cells.

In the invention the Geminin protein, which is to be mutated in order to block DNA replication according to the invention, can be any Geminin of eukaryotes, such as
- the human geminin protein consisting essentially of the amino acid sequence as set forth in SEQ ID NO : 3,
- the murine geminin protein consisting essentially of the amino acid sequence as set forth in SEQ ID NO : 4,
- the Xenopus geminin protein consisting essentially of the amino acid sequence as set forth in SEQ ID NO : 5 or SEQ ID NO : 6,
- the Drosophila geminin protein consisting essentially of the amino acid sequence as set forth in SEQ ID NO : 7,
or any protein having at least 70% identity with said proteins and retaining ability to inhibit DNA replication by inhibiting the complete assembly of the Pre-RC complex.

Advantageously, the invention relates to the use as defined above, wherein said non proteasome-degradable form of the Geminin protein consists essentially of an amino acid molecule consisting of the amino acid sequence as set forth in SEQ ID NO : 3-8, (i.e. SEQ ID NO : 3, SEQ ID NO : 4, SEQ ID NO : 5, SEQ ID NO : 6, SEQ ID NO : 7 or SEQ ID NO : 8) and lacking the following sequence: **RxTLKxzQx.** (SEQ ID NO : 2), wherein x represents any amino acid and z represent a leucine (L) or a valine (V).

The above discloses mutants of Geminin are obtain by substitution of the amino acids R, T, L, K and/or K in as shown in SEQ ID NO : 2 and underlined, such that the degradation by the proteasome pathway is not possible anymore. This means that it is possible to carry out a substitution of 1, 2 3 or 4 of the underlined amino acids.

Another way to inhibit the degradation of geminin protein is to delete the amino acid sequences SEQ ID NO : 2 in the sequence of wild type proteins, for instance as represented in SEQ ID NO : 3-8. In this context, and further a deletion, it is also possible to insert one or more amino acids.

An example of a geminin mutant particularly advantageous in the invention is the mutant consisting essentially of the animo acid sequence as set forth in SEQ ID NO : 9, which is the result of the deletion of SEQ ID NO : 2, followed by an insertion at the same site within the sequence SEQ DI NO : 4. Another example of a geminin mutant particularly advantageous in the invention is the mutant consisting essentially of the animo acid sequence as set forth in SEQ ID NO : 10, which is the result of the deletion of SEQ ID NO : 2, followed by an insertion at the same site, within the sequence SEQ DI NO : 3. Thus, advantageously, the invention relates to use as defined above, wherein said non proteasome-degradable geminin protein consist essentially of the protein as set forth in SEQ ID NO: 9 or 10, or any protein having at least 70% identity and which is not degraded by a cellular degradation process.

The above mentioned inhibitors can be added to a diploid somatic cell by injection of purified protein, or lipofection of protein, or by transfection or infection with a DNA molecule, or a RNA molecule coding for these protein.

Use according to claim 2, wherein said inhibitor is a small interfering molecule inhibiting the expression of said Cdt 1 protein.

As Ctd1 is a target of a non degradable Geminin, for instance as defined above, it is possible to provide inhibitors of Ctd1 to block DNA replication. For instance an inhibition by RNA interference using siRNA, shRNA or miRNA can be carry out. The skilled person could easily determine the best small interfering molecule that can completely block DNA replication.

Advantageously, the invention relates to the use according to the above definition, wherein said haploid eukaryotic somatic cells are able to carry out mitosis. Advantageously, the invention relates to the use according to the above definition, wherein said haploid mammal somatic cells are able to carry out mitosis.

One important point in the process according to the invention, is that the resulting haploid cells, when treated with the inhibitor as defined above, can carry out one or more new complete cell cycle, including DNA replication, when the inhibitor as defined above is removed.

Thus for this reason, it could be advantageous that the inhibitor defined above be "activable" (for instance inducible) when necessary. Activation of the inhibitor may for instance the result of:
- a chemical or physical activation of an inhibitor, for instance an inhibitor achievable by the temperature, pressure, light, etc..,
- a chemical activation of the expression of a mutated protein acting as an inhibitor of the pre-RC (inducible expression system for instance),
or any other means allowing a temporal or time-related control of the inhibitory effect of the inhibitor.

The invention also relates to a protein consisting essentially of the amino acid sequence as set forth in SEQ ID NO : 9 or 10.

These mutant of Geminin are novel.

The invention also relates to a method for producing, in particular *in vitro,* eukaryotic somatic haploid cells from eukaryotic somatic diploid cells, said method comprising a step of contacting eukaryotic somatic diploid cells with an inhibitor of the activity or stability of pre-RC, said inhibitor completely inhibiting the activity or stability of said pre-RC. The method according to the invention may further comprise a step of isolation of the resulting somatic haploid cells, for instance by flow cytometry, by isolating the cells according to their DNA content.

Advantageously, the invention relates to a method for producing, in particular *in vitro,* mammal somatic haploid cells from mammal somatic diploid cells, said method comprising a step of contacting mammal somatic diploid cells with an inhibitor of the activity or stability of pre-RC, said inhibitor completely inhibiting the activity or stability of said pre-RC. The method according to the invention may further comprise a step of isolation of the resulting somatic haploid cells, for instance by flow cytometry, by isolating the cells according to their DNA content.

Advantageously, the invention relates to the method as defined above, wherein said inhibitor is either an inhibitor of the activity or expression of the Cdt1 protein, or said inhibitor is a small interfering molecule inhibiting the expression of said Cdt 1 protein. More advantageously, the invention relates to the method as defined above, wherein said inhibitor is a non degradable form of the Geminin protein, in particular a Geminin protein lacks the following sequence: **RxTLKxzQx.** (SEQ ID NO : 2).

Advantageously, the invention relates to the method as defined above, wherein said cells are mammal somatic cells and wherein said inhibitor is a non-degradable form of the Geminin protein, in particular a Geminin protein lacks the following sequence: **RxTLKxzQx.** (SEQ ID NO : 2).

More advantageously, the invention relates to the method as defined above, wherein the somatic diploid cells are transfected or infected with a DNA or a RNA molecule allowing a conditional expression of a mutated Cdt1 protein, said mutated Cdt1 protein being possibly represented by the amino acid sequence SEQ DI NO : 9 or SEQ ID NO : 10, or any amino acid sequence having at least 70 % identity with said sequences SEQ DI NO : 9 or SEQ ID NO : 10, provided that they have lost their ability to be degraded by the proteasome pathway.

The conditional expression may be the inducible systems Tet-On or Tet-Off well known in the art. The tetracycline-controlled Tet-Off and Tet-On gene expression systems are used to regulate the activity of genes in eukaryotic cells in diverse settings, varying from basic biological research to biotechnology and gene therapy applications. These systems are based on regulatory elements that control the activity of the tetracycline-resistance operon in bacteria. The Tet-Off system allows silencing of gene expression by administration of tetracycline (Tc) or tetracycline-derivatives like doxycycline (dox), whereas the Tet-On system allows activation of gene expression by dox. Since the initial design and construction of the original Tet-system, these bacterium-derived systems have been significantly improved for their function in eukaryotic cells. Tetracycline-controlled gene expression is based upon the mechanism of resistance to tetracycline antibiotic treatment found in gram-negative bacteria. In nature, the Ptet promoter expresses TetR, the repressor, and TetA, the protein that pumps tetracycline antibiotic out of the cell. The difference between Tet-On and Tet-Off is not whether the transactivator turns a gene on or off, as the name might suggest; rather, both proteins activate expression. The difference relates to their respective response to tetracycline or doxycycline (Dox, a more stable tetracycline analogue); Tet-Off activates expression in the absence of Dox, whereas Tet-On activates in the presence of Dox.

The invention also relates to an isolated eukaryotic somatic haploid cell liable to be obtained by the method as defined above.

The invention also relates to an isolated mammal somatic haploid cell liable to be obtained by the method as defined above.

The somatic haploid cells according to the invention are novel, and particularly because they contain a mutated Geminin protein which is not degradable via the proteasome degradation pathway.

The invention relates to an isolated eukaryotic somatic haploid cell provided that said eukaryotic somatic haploid cell is not an embryonic haploid stem cells, said somatic cell being characterized in that it harbors differentiation epigenetic marks, and possibly contain in their genome a sequence coding for a non-degradable Geminin protein.

The invention relates to an isolated plant somatic haploid cell, said somatic cell being characterized in that it harbors differentiation epigenetic marks..

The invention relates to an isolated mammal somatic haploid cell provided that said mammal somatic haploid cell is not an embryonic haploid stem cells, said somatic cell being characterized in that it harbors differentiation epigenetic marks, and possibly contain in their genome a sequence coding for a non-degradable Geminin protein.

The main difference compared to the prior art haploid cells is that the haploid cells of the invention would not be derived from a haploid cell. In the already existing haploid ES cells or in vitro differentiated cells coming from these haploid ES cells, the ES cells come from an oocyte that is not fertilized and activated: so, cells are primarily haploid.

These cells are novel, and particularly, contrary to the prior art, are not originated from a tumor.

In one another advantageous embodiment, the invention relates to isolated mammal somatic haploid cell, said haploid cell deriving from HCT116, DLD1, 3T3 cell lines, or primary fibroblasts.

The invention also relates to a nucleus of an isolated eukaryotic somatic haploid cell as defined above.

The invention also relates to a nucleus of an isolated plant somatic haploid cell as defined above.

The invention also relates to a nucleus of an isolated mammal somatic haploid cell as defined above.

Moreover, the invention relates to a method for carrying out fecundation, in particular *in vitro,* comprising a step of introducing into a female germinal egg a nucleus of an isolated mammal somatic haploid cell as defined above. This method is advantageously carried out provided that it does not intend to provide processes for cloning human beings, or it does not intend to produce human embryos for industrial or commercial purposes.

The method defined above is based on the ability to obtain haploid cells, the nucleus of which could be fused to the nucleus of a recipient germinal female cell.

By this method, it is artificially reproduced the mechanism of fecundation, and could help couples having fertility issues due to a lack of production of gametes.

In one other advantageous aspect, the invention relates to a method for carrying out, advantageously *in vitro,* fertilization comprising a step of introducing into a female germinal cell a nucleus of an isolated plant somatic haploid cell as defined above.

The invention also relates to a method for the production, possibly *in vitro,* of homozygote cells for at least a determined locus, said method comprising a step of genetically modifying at least one locus of a n eukaryotic somatic haploid cell as defined above, a step of inducing DNA replication of said eukaryotic somatic haploid cell and a step of inhibiting cell division.

The invention also relates to a method for the production, possibly *in vitro,* of homozygote cells for at least a determined locus, said method comprising a step of genetically modifying at least one locus of a mammal somatic haploid cell as defined above, a step of inducing DNA replication of said mammal somatic haploid cell and a step of inhibiting cell division.

In the invention, when producing an haploid cell, it could be easy to modify a determined locus within the genome compared to diploid cells, in which it is necessary for some studies to obtain a bialletic modification.

In this context, the genetic manipulation becomes easier, such as the genetics carried out in Yeasts.

According to this method, it is possible to modify the genome of a somatic haploid cells by any molecular biology technic, including CRISP/cas9 technic. When the modification is carried out, it is sufficient to block cell division, by using drugs inhibition the chromosomal segregation, or inhibition of mitosis (such as inhibitor of chromosome segregation). Therefore, when entering to the S phase, the DNA content of the cell will be duplicated, and if the mitosis is not effective, the haploid will become diploid. This cell would have therefore pairs of chromosomes, each member of the pair being strictly identical.

The invention also relates to a nucleic acid molecule coding for a mutated geminin protein, said protein containing a deletion of the sequence **RxTLKxzQx.** (SEQ ID NO : 2), said nucleic acid sequence being possibly contained in a vector allowing the expression of said nucleic acid sequence further to a conditional activation.

More advantageously, the invention relates to a nucleic acid molecule coding for the mutated geminin as set forth in SEQ ID NO : 9 or 10.

More advantageously, the invention relates to a nucleic acid molecule consisting essentially of the nucleic acid molecules as forth in SEQ ID NO : 11 or 12.

The invention will be better understood from the following examples and figures.

### LEGENDE TO THE FIGURES

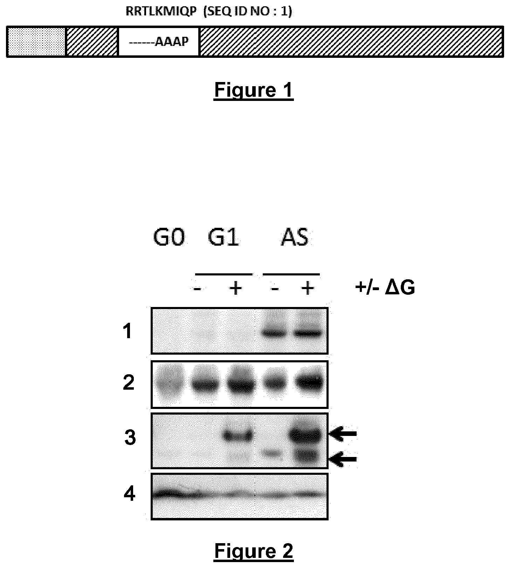

**Figure 1** is a schematic representation of the ΔDboxGeminin protein showing the amino acid sequence (SEQ ID NO : 1) of the destruction box replaced by three alanine residues (in white box). The grey box represents the Flag-Ha tag added at the N-terminus of the protein.
**Figure 2****.** is a western blot showing that the deletion of the destruction box in geminin stabilizes its expression in G1. Analysis by western blotting of total cell extracts from asynchronous (AS) or synchronized in G0 or in G1 3T3 cells after induction or not (+ or - ) of ΔDboxGeminin expression. The G1 phase status of cells was confirmed by CDT1 (2) expression and absence of phosphorylated pRB (1). In G1, endogenous geminin (3; lower band indicated by the arrow) is degraded, but not FLAG-HA-tagged ΔDboxGeminin (3; upper band indicated by the arrow). Histone H3 (4) was used as loading control.

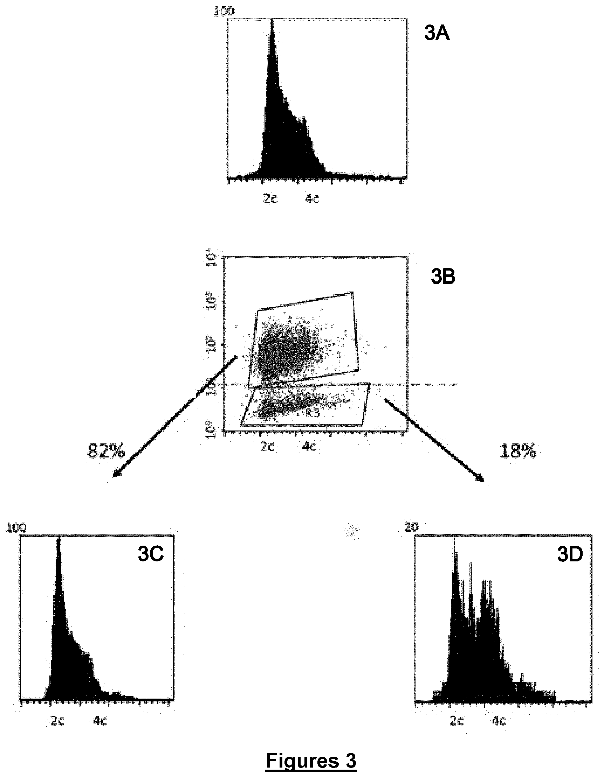

**Figures 3A-D** show cell cycle profile of 3T3 cells synchronized in G0 and then released in the presence of doxycycline to induce ΔDboxGeminin expression for 14 hours. Cells were fixed, incubated with an anti-FLAG antibody (to detect ΔDboxGeminin -positive cells) and stained with propidium iodide (PI; DNA content) and analyzed by flow cytometry.
Figure 3A is a representation of the cytometry results. Y-axis : cell count, and X-axis: DNA content (PI fluorescence).
Figure 3B is a bi-parametric representation of DNA content (PI, X-axis) and FLAG signal (Y-axis).
Figure 3C represent the cell cycle profiles of ΔDboxGeminin -positive 3T3 cells. Y-axis : cell count, and X-axis: DNA content (PI fluorescence).
Figure 3D represent the cell cycle profiles of ΔDboxGeminin -negative 3T3 cells. Y-axis : cell count, and X-axis: DNA content (PI fluorescence).

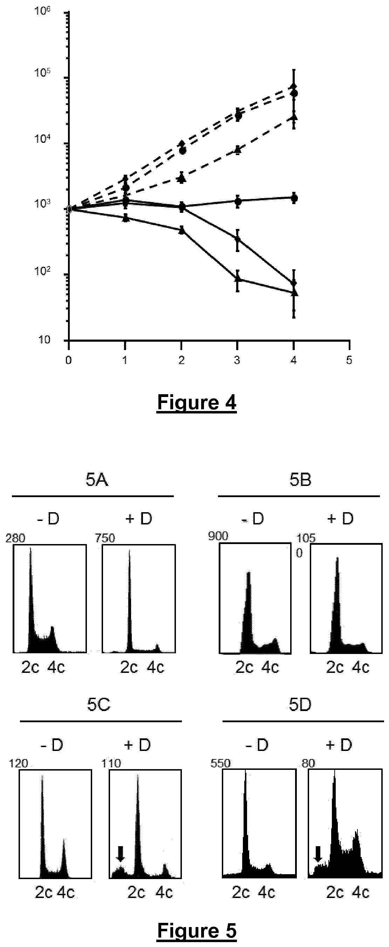

**Figure 4** represents Induction of ΔDboxGeminin expression inhibiting cell proliferation in untransformed somatic cells and killing cancer cells. Cell proliferation curves of 3T3 fibroblasts (3T3; curves with circles), HCT116 colorectal cancer cells (p53+/+) (HCT116) and p53-deficient T98G brain glioblastoma cells (T98G - curves with triangles; curves with diamonds) before (-DOX) and after (+DOX) ΔDboxGeminin induction. Y axis : cell number; X-axis : days. Hatched curves : - Dox; otherwise : + Dox.
**Figures 5A-5D** represent Cell cycle profiles of cell lines 48 hours after or not ΔDboxGeminin expression induction showing the spread profile of the sub-G1 population (pointed by black arrows) in cancer cell lines (HCT116 - 5C and T98G - 5D), but not in non-transformed cells (adult Tail Tip Fibroblasts, TTFs - 5B, and mouse 3T3 fibroblasts - 5A).

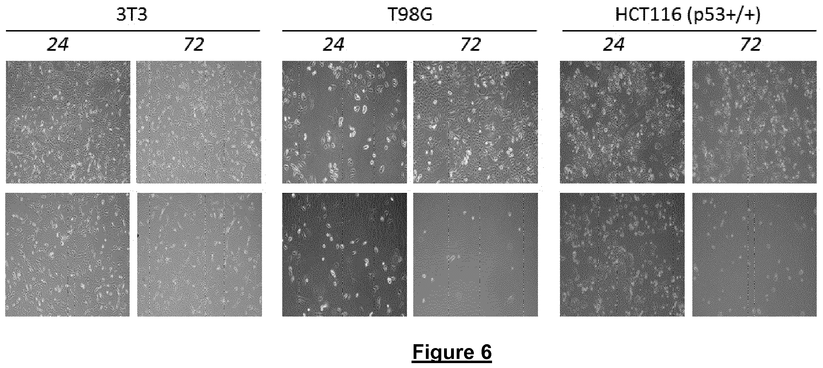

**Figure 6** represents Representative phase contrast images of 3T3, HCT116 and T98G cells 24 hours and 72 hours after induction (lower panels) or not (upper panels) of ΔDboxGeminin showing cell death in cancer cells, but not in 3T3 fibroblasts. Scale bars = 200 µm.

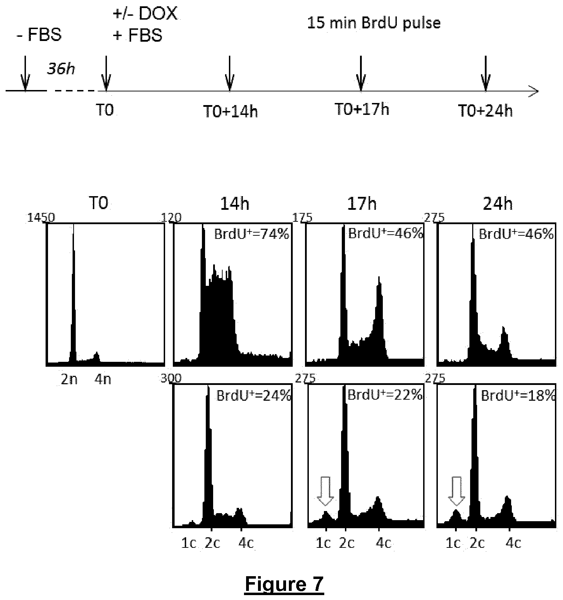

**Figure 7** represents ΔDboxGeminin inhibits DNA replication. Upper panel. Scheme of ΔDboxGeminin induction in 3T3 fibroblasts. Cells were synchronized in G0 (TO) by serum starvation (-FBS) and released by FBS addition in the medium with or without ΔDboxGeminin induction by doxycycline (+/-DOX). Cells were then incubated with BrdU before harvesting. Lower panels. Fluorescent activated cell sorting (FACS) analysis of 3T3 cells incubated or not with doxycycline (+ and -ΔDboxGeminin) after BrdU addition for 15 minutes before collection at 14h, 17h and 24h after release. The x-axis shows the DNA content (propidium iodide staining) and the y-axis the cell number in S-phase, determined by BrdU incorporation and DNA content, is indicated.

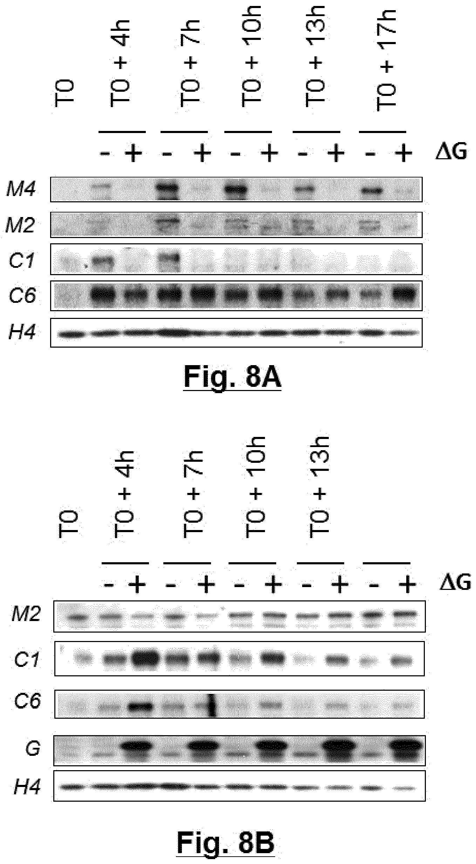

**Figures 8A-B** represent that ΔDboxGeminin prevents DNA replication licensing by blocking CDT1 action. Chromatin-bound (8A) and total proteins (8B) were extracted from 3T3 cells (+ and -ΔDboxGeminin (noted G) induction) at different time points after release from G0 and analyzed by western blotting. CDT1 binding to chromatin was inhibited by ΔDboxGeminin expression, but not its accumulation in total extracts, confirming that CDT1 degradation is dependent on DNA replication and not on cell cycle progression. MCM2 (M2) and MCM4 (M4) binding to chromatin was also inhibited, as expected in cells with a licensing reaction defect. Conversely, CDC6 (C6) binding to chromatin was not affected, possibly because it occurs before and independently of CDT1 (C1). Histone H4 (H4) is used as loading control.

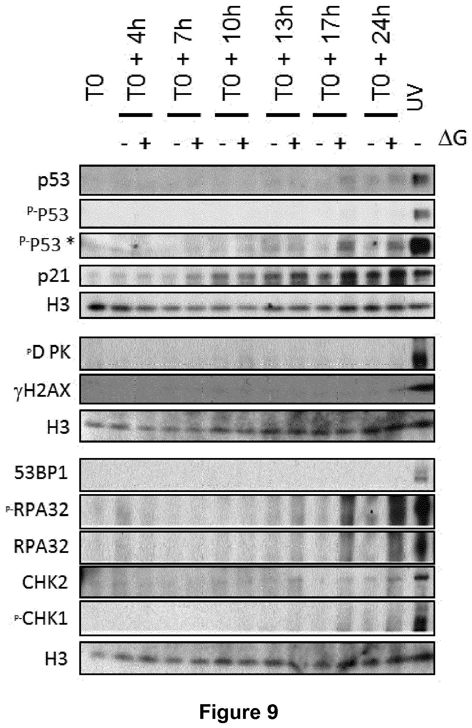

**Figure 9** represents Licensing inhibition by ΔDboxGeminin does not induce checkpoint activation during the first cell cycle. Different checkpoint proteins were analyzed by western blotting after licensing inhibition. As a positive control, 3T3 cells were exposed to UV to confirm that checkpoints are functional in these cells.

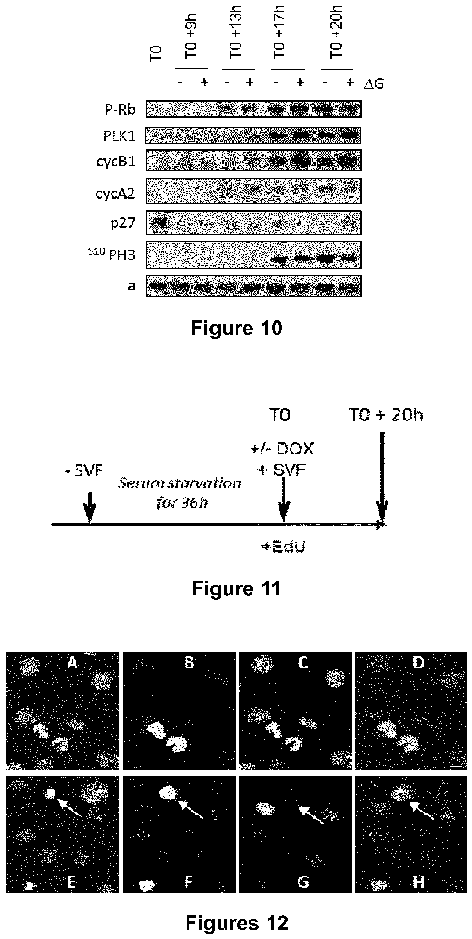

**Figure 10** represents that ΔDboxGeminin expression does not prevent the expression of mitotic markers. Total protein extracts from 3T3 cells were analyzed by western blotting at different time points after release from quiescence (G0 =T0) and ΔDboxGeminin induction (+) or not (-).
**Figure 11** is a scheme showing how to manage the expression of ΔDboxGeminin to induce mitotic entry of EdU-negative cells. 3T3 cells were synchronized in G0 and then released in the presence of EdU (and + or- DOX to induce ΔDboxGeminin). At T0+20h, cells were fixed to assess EdU incorporation and and stained for different markers.
**Figures12A-H** show that in control cultures (-ΔDboxGeminin - 12A-D) all mitotic cells were EdU-positive (i.e., postreplicative cells). By contrast, in +ΔDboxGeminin cultures (12E-H), some unlicensed mitotic cells without detectable EdU staining could be observed (red arrow). Scale bars = 5 µm. A and E : DAPI ; B and F : PH3 ; C and G : EdU and D and H : Merge.

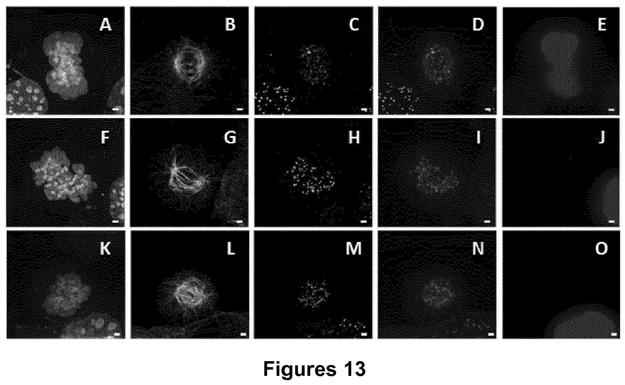

**Figures 13A-O** are Super-resolution microscopy analysis of fixed 3T3 cells incubated with anti-αtubulin (B, G and L) and -kinetochores (CREST ; C, H and M) antibodies and stained with EdU (E, Ja,d O) 17 hours after release from G0 in the presence of EdU and with or without doxycycline to induce (F-O) or not (A-E) ΔDboxGeminin expression (+ and -ΔDboxGem). Representative images of one non-induced control mitotic cell (upper panels 1-E) and one unlicensed mitotic cell (lower panels K-O) with bipolar spindle despite the absence of DNA replication. Scale bars = 1 µm. A; F, E : DNA labelling and D, I and N : merge.

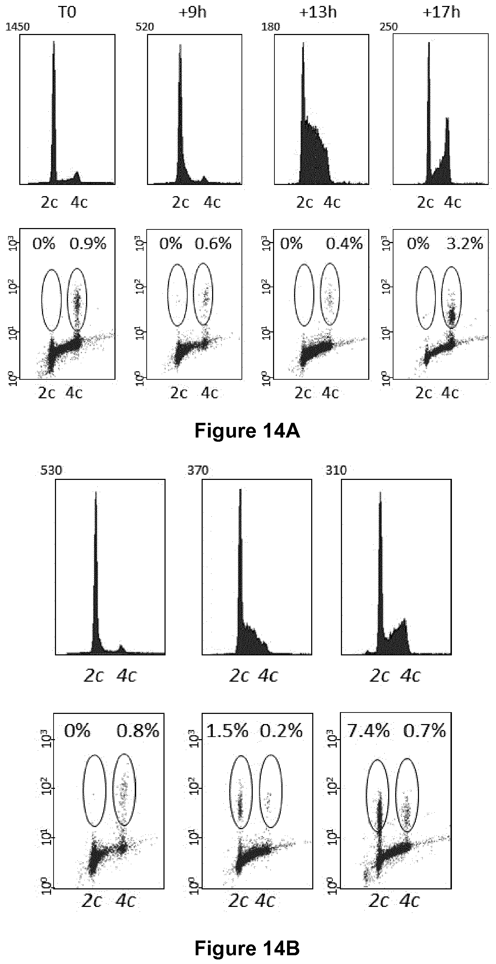

**Figures 14A-B** **:** ΔDboxGeminin expression leads to mitotic entry of 2C DNA content cells. The lower panels show the ^{ser10}PH3 signal in cells with different DNA content (2C: pre-replicative cells; and 4C: post-replicative cells) assessed by flow cytometry at different times after release from quiescence in non-induced (-DOX ; 14A, upper panels) or ΔDboxGem-induced 3T3 cells (+DOX, 14 B, lower panels). DNA content was assessed by propidium iodide staining and the percentage of 2C and 4C cells is indicated for each condition.

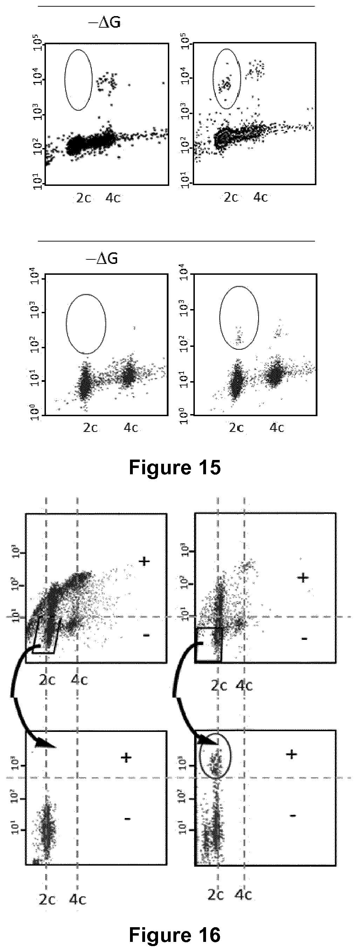

**Figure 15** shows mouse adult tail-tip fibroblasts (upper panels) and embryonic fibroblasts (MEFs) (lower panels) were infected with ΔDboxGeminin-encoding viruses, synchronized in G0 and released in the cell cycle. The DNA content and ^{ser10}PH3 level were measured by flow cytometry 20 hours after release and induction or not of DDboxGeminin expression (+ and - ΔDboxGem). Black circles highlight the ^{ser10}PH3-positive 2C cell populations.
**Figure 16** shows simultaneous three-color FACS analyses of 3T3 cells that express ΔDboxGeminin (right panels, +DOX) or not (left panels, -DOX). Bi-parametric representation of EdU detection in 2C and 4C cells. EdU-negative 2C cells were then gated to assess ^{ser10}PH3 staining. The circle highlights the EdU-negative and ^{ser10}PH3-positive 2C cell population observed only upon induction of ΔDboxGeminin expression.

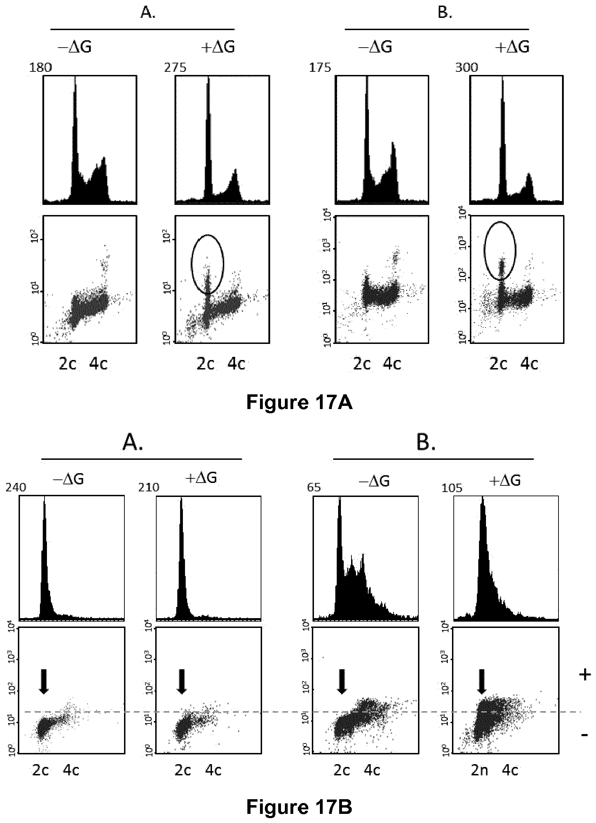

**Figure 17A****:** 3T3 cells were synchronized in G0 and released with or without (+ and - ΔDbox) induction of ΔDboxGeminin expression for 20 hours. Cells were then fixed and the expression of ^{ser10}PH3 or MPM2 (a mitosis-specific phosphorylated epitope) was analyzed by flow cytometry to confirm the presence of mitotic cells with 2C DNA content (propidium iodide; PI) after induction of ΔDboxGeminin expression (highlighted by black circles).
**Figure 17B** **:** Cyclin B1 and DNA content were analyzed by flow cytometry in synchronized 3T3 cells at T1 and T2 (7 hours and 17 hours post-release and ΔDboxGeminin induction, respectively). Similarly to ^{ser10}PH3 and MPM2, cyclin B1 accumulated in 2C cells (pointed by the black arrow) at T2 after ΔDboxGeminin induction.

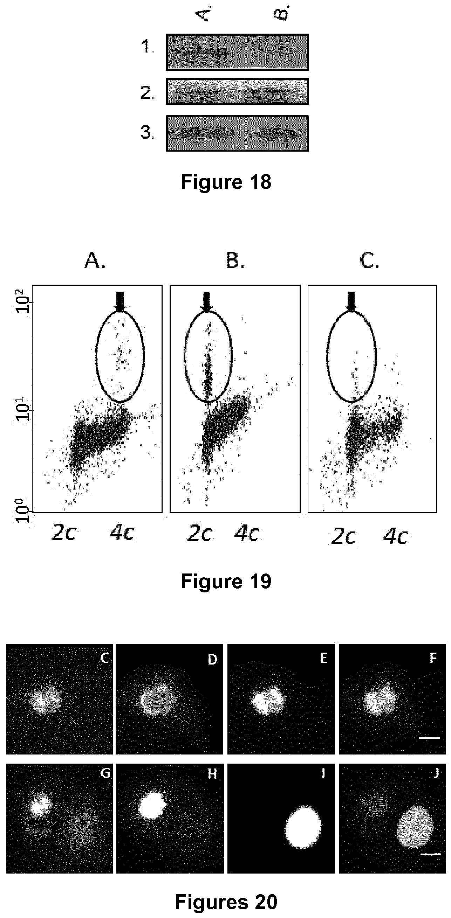

**Figure 18 -20** : 3T3 cells were infected with plasmids encoding shRNAs against GFP (control, A.) or CDT1 (shCDT1, B.), synchronized in G0 and then released in the cell cycle.
**Figure 18** is a western blot where total protein extracts were analyzed by western blotting to assess the efficiency of CDT1 silencing (left panel)
**Figure 19** shows that, at 20 hours post-release, cells were fixed and ^{ser10}PH3 level and DNA content (propidium iodide staining) were analyzed by flow cytometry.
**Figures 20C-J** shows that the mitotic status of ser10PH3 positive/EdU negative cells, shCDT1-expressing 3T3 cells were synchronized in G0 and released in the presence of EdU for 20 hours, fixed and incubated with an anti-^{ser10}PH3 antibody to detect by immunofluorescence the presence of EdU-negative cells in the G2/M phase (=unlicensed mitotic cells, right panel). Scale bars = 5 µm. C and G : DAPI; D and H : ^{ser10}PH3 ; E and J : merge.

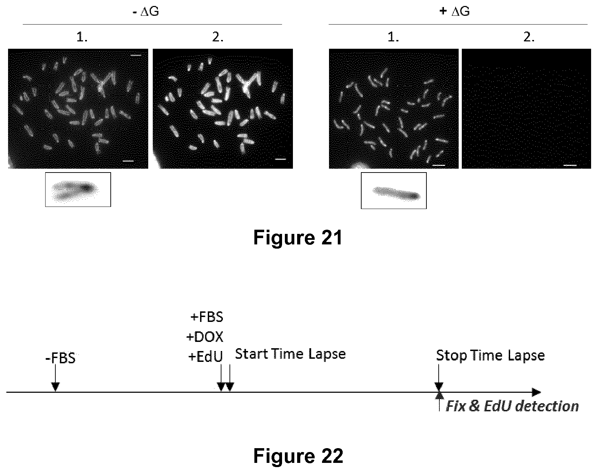

**Figure 21** shows that MEFs that express inducible ΔDboxGeminin were synchronized in G0 and released in the presence of EdU with (+ΔDboxGem) or without (-DDboxGem) DOX. After 12 hours, cells were incubated with colcemid for 4 hours and EdU incorporation was assessed in metaphase spreads. Control metaphase spreads (-ΔDboxGem) (left panels) had 40 chromosomes with two replicated chromatids attached by the kinetochore. By contrast, some +ΔDboxGem metaphase spreads (example in right panels) were EdU-negative and had 40 chromosomes with single chromatids (see enlarged inset). Scale bars = 8 µm.
**Figure 22** shows a schematic of the experiment to investigate the fate of unlicensed (EdU-negative) mitotic cells. 3T3 cells that transiently express GFP-H2B were synchronized in G0 and released in the presence of EdU and DOX to induce DDboxGeminin expression. At the end of the time lapse microscopy analysis, cells were fixed and processed for EdU detection.

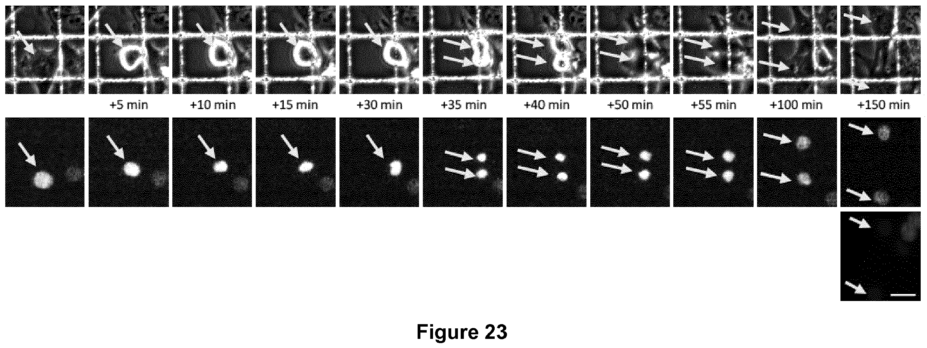

**Figure 23** shows that unlicensed cells can divide without DNA replication. Images from the time lapse experiments described in C. Arrows show a mitotic cell and then its two daughter cells. The right panels show that the two daughter cells were EdU-negative. Scale bars = 25 µm.

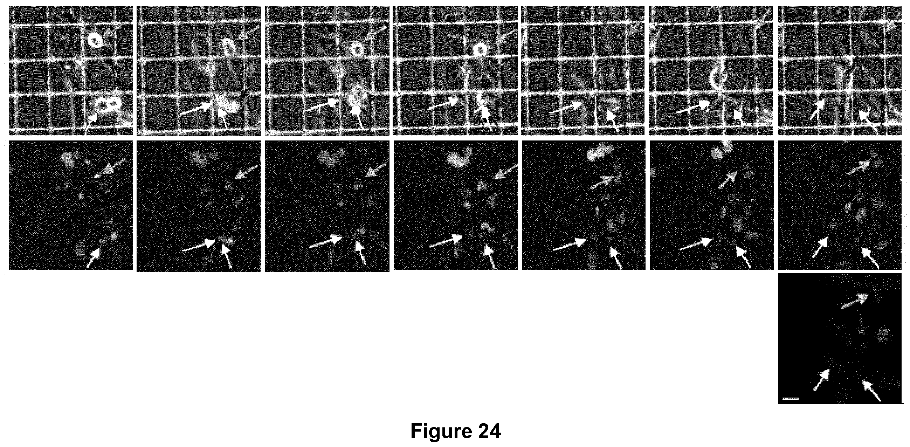

**Figure 24** represents Images of a time lapse experiment showing the different fates of unlicensed mitotic cells: i) completion of cell division (arrows); ii) formation of a binucleated cell (cytokinesis failure) and iii) formation of a mononucleated cell (mitotic slippage) Scale bars = 25 µm.

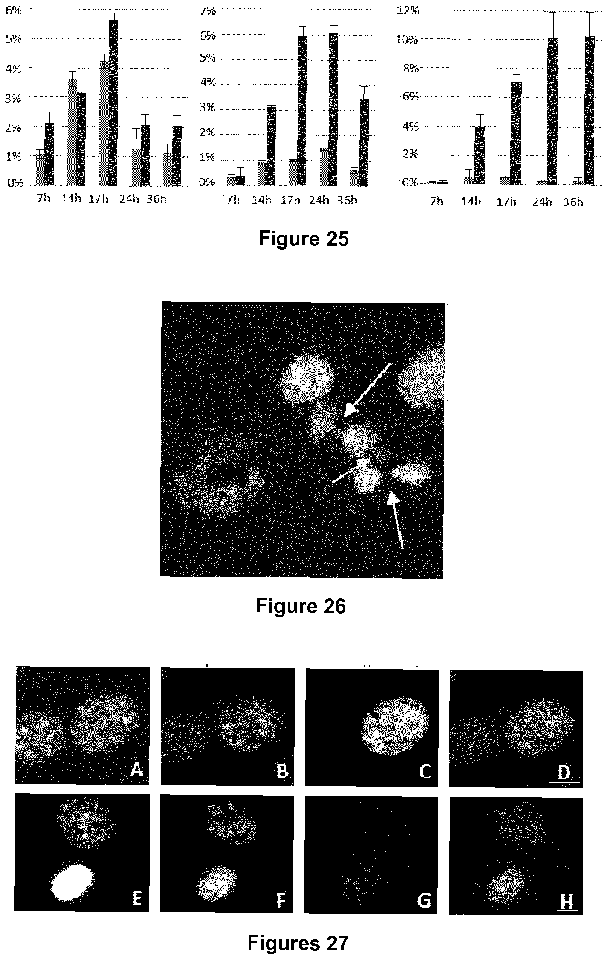

**Figure 25** represents histograms showing at different time points after release from G0, 3T3 cells that were fixed and stained with DAPI (to determine the proportion of anaphase bridges and micronuclei) and with an anti-^{ser10}PH3 antibody (to determine the number of mitotic cells by immunofluorescence). Anaphase bridges and micronuclei started to appear after the first wave of mitoses (from 14h post-release), suggesting that they might result from mitotic failure. + and -DOX, doxycycline induction or not, respectively, of ΔDboxGeminin expression (data are the mean ± SEM of nine fields of view each from three experiments).
**Figure 26** is a picture representing expression of ΔDboxGeminin in 3T3 cells that induces anaphase bridges (white arrows) and micronuclei (yellow arrow) after the first mitotic wave (DAPI staining at 21 h after release from GO). Scale bars = 5 µm.
**Figures 27A-H** represents photos where 3T3 cells were synchronized in G0 and then incubated with doxycycline (+ΔDboxGem ; E-H) or not (-ΔDboxGem ; A-D) for 24 hours. Then, cells were incubated with EdU for 15min, fixed and stained for gH2AX, EdU and DAPI. In some cells, gH2AX signal, mainly restricted to DAPI-negative nuclear structures, could be observed, suggesting the nucleolar localization of damaged DNA. None of these cells could replicate as incorporation of BrdU was never detected in these cells The nucleolar gH2AX signal was weaker and differently distributed in the ΔDboxGem+ cells (white arrow) compared with the replicating control cells or induced cells that likely do not efficiently express ΔDboxGeminin upon induction (yellow arrow) and therefore show canonical DNA damages induced by basal replicative stress. Scale bars = 5 µm. Aand E : DAPI, B and F : γH2AX, C and G : EdU (pulse and D and H : Merge.

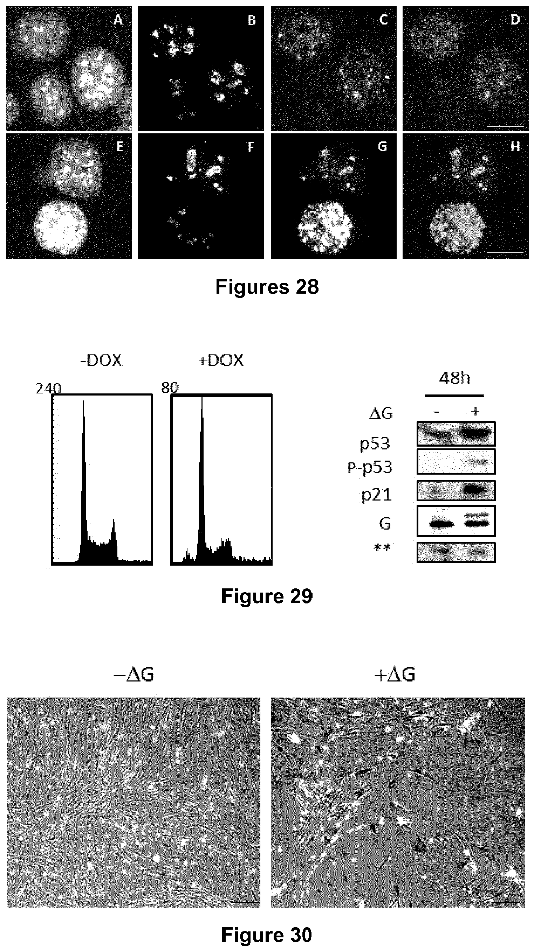

**Figures 28A****-H:** 24 hours after release from G0 and incubation with doxycycline (+ΔDboxGeminin ; A-D) or not (-ΔDboxGeminin ; E-H), 3T3 cells were fixed and the expression of γH2AX (B and F) and nucleolin (a marker of nucleoli; C and G) was assessed by immunofluorescence. Scale bars = 5 µm. A and E : DAPI and D and F : merge.
**Figure 29** **:** 48 hours after release from G0 and incubation with doxycycline or not, cell cycle distribution was analyzed by flow cytometry and total protein extracts by western blotting. Upon ΔDboxGeminin expression, the number of 3T3 cells with 2C DNA content increased and p53 and p21 were induced,
**Figure 30** **:** 10 days after ΔDboxGeminin induction or not (+ and -), TTFs were fixed and stained for β-galactosidase activity, a marker of premature senescence. Scale bars = 20 µm.

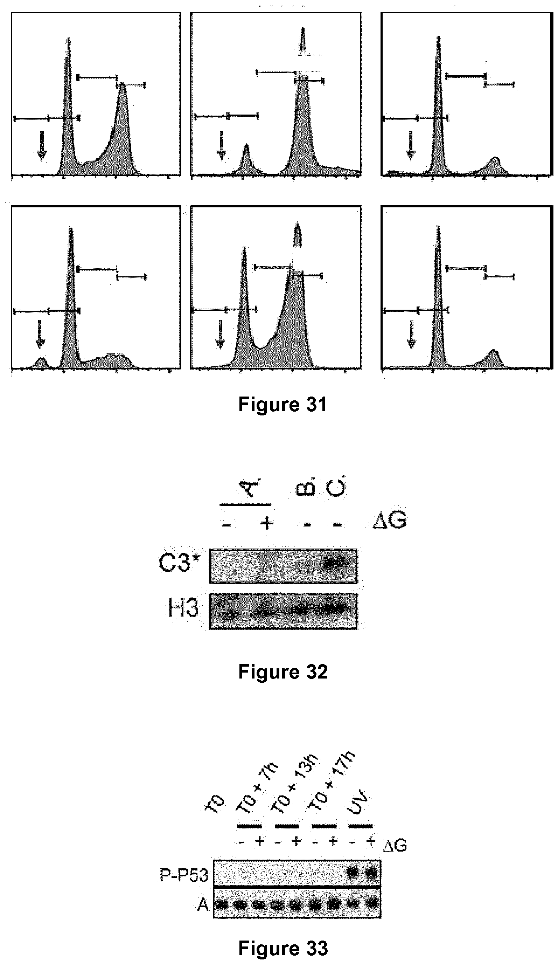

**Figure 31** represents FACS profiles showing the DNA content (propidium iodide staining) of 3T3 cells 20 hours after release from G0 in the presence (+ΔDboxGem) or not (-ΔDboxGem) of doxycycline alone (CTRL - first column) or combined with RO-3306 treatment (a CDK1 inhibitor that prevents mitotic entry second column) or after UV treatment (performed upon release to induce DNA damage; third column). The sub-G1 population (arrow) observed in ΔDboxGem-expressing cells (bottom left) showed a discrete and relatively sharp profile. RO-3306 prevented mitotic entry and the appearance of the 1C DNA cell population (middle bottom panel). UV treatment blocked cell cycle progression in -ΔDboxGem cells (top right) and induced apoptosis, as indicated by the spread subG1 population observed by FACS and confirmed by cleaved caspase 3 induction visualized by western blotting of total protein extracts
**Figure 32** is a control western blot used as control of the experiment of figure 31. A short incubation with hydroxyurea (HU) on synchronized cells for 20 hours was carried out to block DNA replication did not induce detectable apoptosis. A: next G1, B: HU and C : U.V. C3* : cleaved caspase 3 and H3 : histone H3.
**Figure 33** is a western blot showing that ΔDboxGeminin expression does not induce p53 activation (phosphorylation at serine 15), but does not inhibit its activation after UV-induced DNA damage.

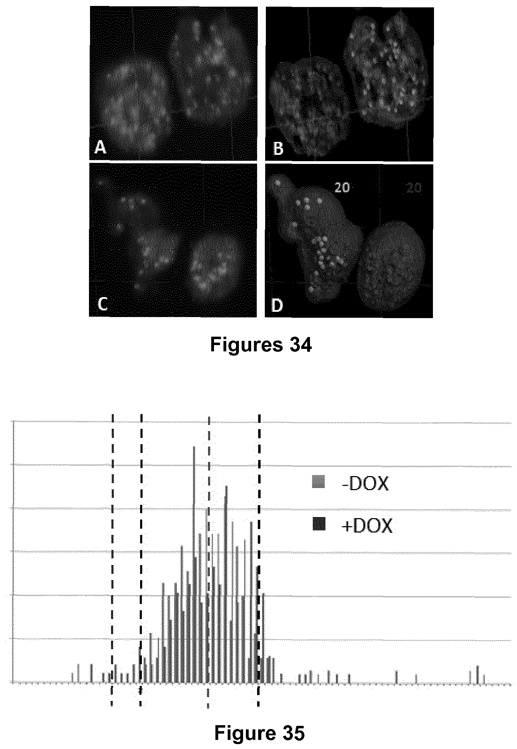

**Figures 34A-D** **:** After ΔDboxGeminin expression induction (lower panels, +DOX) or not (upper panel, CTRL), mouse ES cells were fixed, incubated with an antibody against kinetochores (CREST) and then stained with DAPI (DNA). The number of CRESTpositive dots per nucleus was determined with the IMARIS software after 3D image reconstruction and is indicated in the images.
**Figure 35** **:** Number of CREST dots per nucleus of mouse embryonic stem cells in which ΔDboxGeminin expression was induced (red bars) or not (blue bars; control). Black dashed lines highlight the theoretical number of kinetochores per nucleus in 2C and 1C cells. The blue dashed line represents the average number of CREST dots observed in control cells to visualize the approach bias. The dashed line indicates the average CREST dots observed in cells with less than 20 dots. With this approach, 20% of kinetochores were not detected in control cells. In the ΔDboxGeminin expressing cell population with less than 20 dots, the average number of CREST dots was 15.7. This value would correspond to 20 kinetochores when the bias observed in control cells is taken into account.

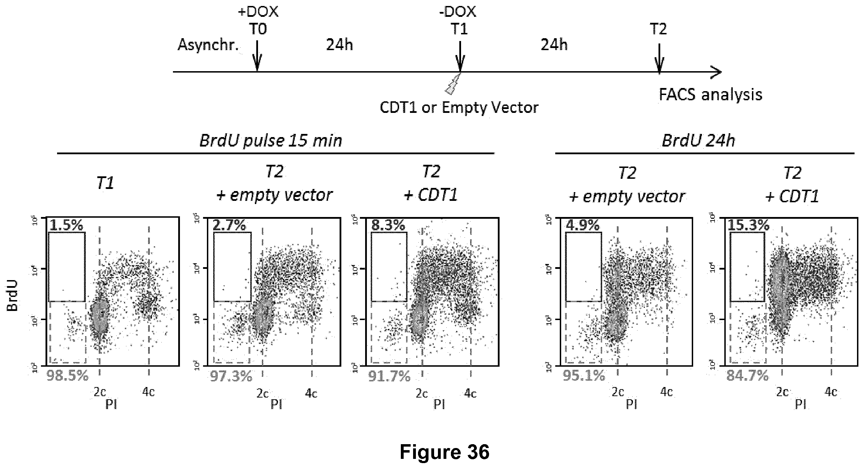

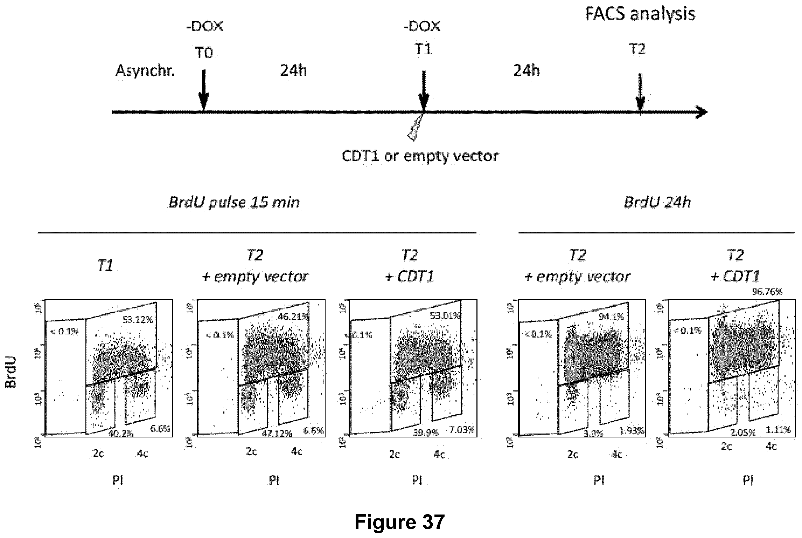

**Figure 36** **:** The 1C cell population can re-enter the cell cycle after CDT1 overexpression. At T0, ΔDboxGeminin expression was induced (+DOX) in asynchronous 3T3 cells for 24h and then DOX was washed off and cells were transfected with a CDT1-encoding plasmid or empty vector (T1). Cells were incubated with BrdU for 15min before fixation at T2 (left panels) or for 24 hours before analysis at T2 (right panels). During the last 24 hours, 15.3% of 1C cells transfected with CDT1 incorporated BrdU whereas 4.9% did after empty vector transfection. Upon CDT1 overexpression, 8.3% of 1C cells were actively replicating DNA (BrdU+) during the 15 minutes preceding the fixation, compared with 2.7% of cells transfected with empty vector. For each condition, the percentage of BrdU+ (red) and BrdU- (blue) cells in the sub-G1 population are shown. The same experiment with non-induced (-DOX) cells is shown in **Figure 37****.**
**Figure 37** **:** top panel represents a scheme of the experiment related to Figure 6F for cells in which ΔDboxGeminin expression was not induced (-DOX) at T0.
   Lower panel represents a bi-parametric analysis images of DNA content (propidium iodide; PI) and BrdU incorporation in non-induced 3T3 cells that were transfected with empty vector or the CDT1-encoding plasmid at T1 and incubated with BrdU for 15 minutes prior to harvesting at T2 (to visualize replicating cells, 24 hours after transfection) or for 24 hours (to quantify the proportion of cells that have replicated DNA during the last 24 hours). Cells were fixed and stained for DNA (PI) and BrdU incorporation assessed.
   Results show that compared to vector alone, CDT1 overexpression did not dramatically change the cell cycle features of 3T3 cells in which ΔDboxGeminin was not induced.

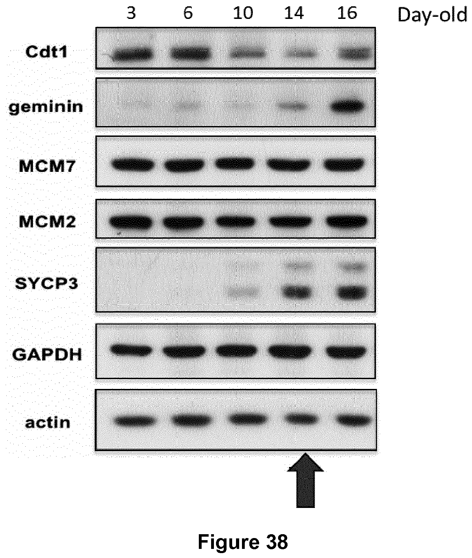

**Figure 38** represents a western blot where testes were dissected from Sv129/B6 mice at different days post-partum and the expression of the indicated proteins was assessed by western blot analysis. Actin and GAPDH were used as loading controls and SYCP3 to monitor meiosis onset.

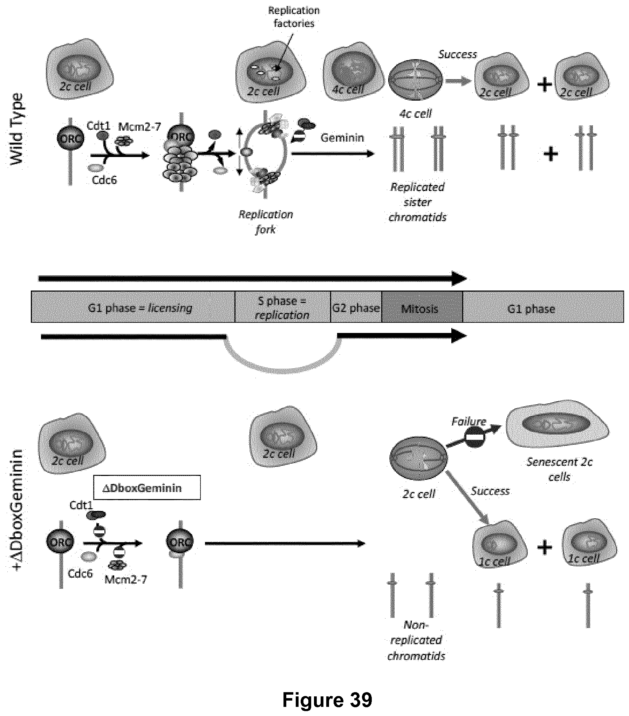

**Figure 39** **:** Upper part: Scheme of temporal coordination between DNA replication and cell division in wild-type diploid cells (2C chromosomes). During G1 phase, the licensing reaction takes place, resulting in the binding of the DNA helicase MCM2-7 at the DNA origins. This binding is dependent on the presence of the proteins ORC, CDC6 and CDT1. Upon S-phase entry, replication origins are activated and DNA is replicated.
   Geminin starts to be synthesized and inhibits further binding of CDT1 onto origins, preventing re-licensing and re-replication of DNA. When DNA replication is completed, cells enter into mitosis with 4C chromosomes, the two replicated sister chromatids remaining attached by the centromere until anaphase onset.
   Lower panel: fates of normal cells in which licensing is inhibited by overexpression of ΔDboxGeminin. The absence of the Dbox (destruction box) stabilizes Geminin, normally degraded in G1 by the action of the APC/C. The ΔDboxGeminin prevents binding of CDT1 onto origins and inhibits formation of the pre-RCs. Unlicensed cells enter into mitosis with unreplicated chromatids (2C chromosomes). The majority of these mitoses fail to complete, generating one single cell with one (mitotic slippage) or two (cytokinesis failure) nuclei. These cells display nucleolar γH2AX staining and enter into senescence. A discrete population of unlicensed cells manages to divide and gives rise to two daughter cells, some of them containing 1C chromosomes.

### EXAMPLES

### Example 1: Materials and Methods

### Plasmids and constructs

Deletion of the destruction box (Dbox) and addition of N-terminal Flag-HA epitopes were performed by PCR. ΔDboxGeminin was finally cloned into pTRIPZ using the Age1-Mlu1 cloning sites (Supplementary Information 1). The plasmid encoding the shRNA against CDT1 was from Sigma-Aldrich (TRCN0000174484). The CDT1-pCDNA3 plasmid is described in Coulombe et al. [Coulombe et al. 2013, Nature Communications 4,pp 1-10].

### Preparation of viral particles and infection

HIV-derived vectors pseudo-typed with the VSV-G envelope protein were produced by transient co-transfection of the Gag-Pol packaging construct psPAX2, the envelop plasmid pMD.2G and the self-inactivating (SIN) HIV-1-derived vector coding for the ΔDboxGeminin or shRNA of interest. 36 hours after transfection, particles were harvested and filtered before infection of different cells.

### Cells and media

Mouse embryonic fibroblasts (MEFs) were derived as previously described [Ganier et al. Proc Natl Acad Sci U S A. 2011 Oct 18;108(42):17331-6]. Briefly, 13.5-day-post-coitum wild type C57BL6 mouse embryos were dissected and gonads, internal organs and head were removed before MEF isolation. Tissues were then dissociated with 0.05% trypsin/EDTA (Gibco, Invitrogen) at 37°C for 15 minutes to isolates single cells. To obtain tail tip fibroblasts, tail tips from adult mice were sliced into small pieces, trypsinized and plated to derive fibroblast cultures. All fibroblasts were expanded for three passages in Dulbecco's Modified Eagle Medium (DMEM) (Invitrogen) with 10% fetal bovine serum (FBS) (catalog no. S1810; Biowest) before being used for experiments.

3T3, TTF, MEFs and T98G cells were grown in high-glucose DMEM (Invitrogen) supplemented with 10% FBS, 2mM L-glutamine (Invitrogen) and 1 mM sodium pyruvate (Sigma). HCT116 p53^{+/+} cells were grown in McCoy's 5a medium modified with 10% FBS. The mouse ES cell line CGR8 was from C. Crozet (Institut de Génétique Humaine, Montpellier, France) and was cultured as described in Ganier et al. [Ganier et al. Proc Natl Acad Sci U S A. 2011 Oct 18;108(42):17331-6]. Specifically, ES cells were grown on 0.1 % gelatin without feeders in ES cell medium [Glasgow minimum essential medium (Invitrogen) with 10% FBS, 0.1 mM β-mercaptoethanol, 1mM sodium pyruvate, 1% nonessential amino acids (Gibco), 2mM L-glutamine and 1,000 U/mL Leukemia inhibitory factor (LIF) (ES-GRO)] at 37 °C in 5% CO₂.

To obtain stable cell lines that express inducible ΔDboxGeminin, exponentially growing cells were infected with HIV-derived viral particles that were previously filtered and concentrated by ultracentrifugation. 48 hours after infection, cells were selected with 2µg.mL⁻¹ puromycin. For ES cells, clonal selection was performed to ensure ΔDboxGeminin expression upon doxycycline induction.

Primary TTFs and MEFs were infected at sub-confluence with viral particles containing pTRIPZ-ΔDboxGeminin and processed immediately for experiments.

Expression of ΔDboxGeminin was induced by adding 1-2µg.mL⁻¹ doxycycline (DOX, Clontech) in the medium.

### Immunofluorescence

Cells were plated on coverslips, washed twice in PBS and then fixed in 3% paraformaldehyde (PFA) at room temperature (RT) for 10min, washed with PBS, and permeabilized with PBS/0.2% Triton X-100 for 5min except for CREST and γ-tubulin staining for which cells were fixed in cold Methanol for 5min. Then, cells were washed three times in PBS/2% BSA for 10min, incubated with primary antibodies diluted in blocking buffer (PBS/0.5%Tween/2%BSA) at 4°C overnight, except for the CREST antibody (diluted in PBS/0.5%Tween/5% non-fat milk and added at RT for 1 hour). After, three washes in PBS/0.5%Tween, cells were incubated with secondary antibodies diluted in blocking buffer for 1 hour and DNA stained with Hoechst. Cells were mounted on glass slides with Prolong (Sigma-Aldrich). For co-staining with EdU, EdU was detected prior to immune-detection according to the manufacturer's instructions.

Stainings were observed using widefield fluorescence microscopes (Leica, Germany and Carl Zeiss, Germany) and 63x 1.4NA PL APO lenses. Filter sets allowing quadruple staining were used (Carl Zeiss FS49, FS38HE, FS43 and FS50). Images were acquired using a Hamamatsu ORCA Flash4 sCMOS camera. For chromosomes counting using CREST staining, ES cells were fixed and stained as described above for EdU and CREST 24 hours after ΔDboxGeminin induction. Images were acquired with the Zeiss Axioimager Z3 Apotome and stacks were acquired according to the Nyquist criterion. For better contrast, Grid Projection Illumination Microscopy (aka Apotome) was used. 3D reconstruction was performed using Imaris software (Bitplane, Oxford Instruments). Briefly the nuclear envelope was modelized using isosurface detection and CREST foci were detected using spot detection. Number of spots/envelope were automatically derived using the split into surface object Xtension.

3D-SIM imaging was performed using an OMX-V3 microscope (General Electrics) equipped with 405 nm, 488 nm and 561 nm lasers and the corresponding dichroic and filter sets. Far Red channel, used to detect EdU incorporation, was acquired in widefield mode only. Reconstruction and alignment of the 3D-SIM images was carried out with softWoRx v 5.0 (General Electrics). 100nm green fluorescent beads (Life Technologies) were used to measure the optical transfer function (o.t.f.) used for the 405 and 488 channels, and 100 nm red fluorescent beads (Life Technologies) were used to measure the o.t.f. used for the 561 channel. 170 nm TetraSpeck beads (Life Technologies) were employed to measure the offsets and rotation parameters used in the image registration. Reconstructed 3D-SIM images were analyzed using Image J 1.4.7v software.

### Metaphase spreads

pTRIPZ-ΔDboxGeminin-infected MEFs were synchronized in G0 by confluence and then released by splitting them in fresh medium containing 15% FBS + 10 µM EdU with or without DOX. 15 hours later, 100nM Karyomax was added in the medium for 7 hours. Then, metaphase spreads were prepared as described in Eot-Houllier et al. [Eot-Houllier et al Genes Dev. 2008 Oct 1;22(19):2639-44], fixed and processed for EdU detection and DNA staining.

### Videomicroscopy

For video microscopy experiments, ΔDboxGeminin 3T3 cells were infected with viruses encoding H2B-GFP and processed for time lapse experiments without selection. 3T3 cells were seeded on µ-Dish 35 mm, high Grid-50 Glass Bottom gridded coverslips and were synchronized by serum starvation, as described above, and released in the presence of 10µM EdU and DOX or not. Tilescan Timelapse images were acquired every 5 minutes for 20h. To ensure a large sample size, the four, whole quadrants of the Ibidi dish were recorded, then fixed and processed for EdU detection as described above.

### Flow Cytometry

For PI, BrdU/PI and ^{ser10}PH3/PI experiments, cells were fixed in cold 70% Ethanol/PBS and then processed for indirect immunofluorescence, as described above. To detect cyclin B1/PI, MPM2/PI and FLAG/PI, cells were fixed in 3% PFA in PBS at RT for 15min, permeabilized in 0.5% Triton X-100 for 5min and processed for indirect immunofluorescence. For DNA staining, cells were treated with 50 µg.mL⁻¹ RNase A (Sigma, R6513) and stained with 25 µg.mL⁻¹ propidium iodide.

For BrdU detection, cells were incubated for the indicated times before harvesting and fixation. Then, cells were treated with 2N HCl for 30min and washed in PBS 0.2% Tween (PBS-T) with 5% BSA before BrdU detection.

For EdU/^{ser10}PH3/DRAQ5 detection, fixed cells were first processed for EdU detection following the manufacturer's instructions, and then incubated with the anti-^{ser10}PH3 antibody in PBS-T 5% BSA at 4°C overnight. Cells were then washed twice in PBS-T and incubated at RT with the anti-rabbit PE antibody (1/200 in PBS-T/5% BSA) and after two washes in PBS-T, stained with 2.5 µm DRAQ5 (DNA) and 50 µg.mL⁻¹ RNase A at RT for 1 hour.

Cells were analyzed with a FACSCalibur flow cytometer using the CellQuestPro software and a Miltenyi MACS quant cytometer.

### Cell synchronization and treatments

To synchronize 3T3 cells in G0/G1, cells were incubated in serum-depleted medium (0.5% FBS) for 36 hours. Cells were then released in medium supplemented with 15% FBS +/- DOX and +/- 10µM EdU. Primary TTFs and MEFs were synchronized by confluence 16 hours after infection and released by splitting them (1:4 ratio).

To express CDT1, 3T3 cells were transfected with 5µg of plasmid pCDNA3-CDT1 or empty vector using Nucleofector TN II ; Amaxa Biosystem ; AAD-1001N. To induce DNA damage, synchronized cells were exposed to UV (0.002 joules) using the Bioblock Scientific BLX-254 upon release or incubated with 2mM hydroxyurea for 20 hours. To block mitotic progression, 9µM RO-3306 was added in the medium upon G0/G1 release.

### Cell proliferation curves:

At day 0, 10³ cells (3T3, T98G, HCT116) were seeded at sub-confluence and then harvested every day for cell counting. Values represent the average of three experiments ± SD.

### Senescence-associated β-Galactosidase staining

Sub-confluent asynchronous mouse TTFs were infected with ADboxGeminin-encoding viral particles and cultured in the presence, or not, of DOX for 14 days. Senescence-associated β-galactosidase (SA-β-gal) activity was assessed as described *[*Debacq-Chainiaux et al., 2009, Nature Protocols 4, 1798 - 1806]. Briefly, cells were washed twice in PBS, fixed in 2% formaldehyde-0.2% glutaraldehyde at RT for 5min, washed twice with PBS and incubated at 37°C in freshly prepared SA-β-gal stain solution (1 mg.mL⁻¹ of X-gal; 40mM citric acid; sodium phosphate, pH 6.0; 5mM K₄[Fe(CN)₆] 3H2O, 5mM K₃[Fe(CN)₆]; 150mM NaCl and 2mM MgCl₂) overnight. Cells were washed with PBS, fixed with methanol and air-dried before analysis.

### Western blotting

To prepare total cell extracts, cells were harvested, washed twice with PBS, lysed in 2x Laemmli buffer, sonicated (3x30 seconds) and boiled for 5min. To obtain chromatin-enriched fractions, a protocol adapted from Lutzmann et al. [Lutzmann et al. 2012 Molecular Cell, 47, 523-534] was used. Briefly, cells were harvested, washed with PBS and lysed on ice in CSK buffer (150mM NaCl, 10mM HEPES pH 7.5, 300mM Sucrose, 1 mM MgCl₂, 1mM EDTA, 1mM ATP·MgCl₂, 1 mM DTT, 0.5% Triton X-100, phosphatase-inhibitors [Calbiochem], the protease inhibitors leupeptin, aprotinin, and pepstatin at a final concentration of 10µg/mL) for 30min. Lysed cells were then centrifuged at 3800g, 4°C for 5min to obtain the soluble fraction. Pellets were washed twice with CSK buffer on ice for 5min, centrifuged at 3800g, 4°C for 5min, solubilized in 2x Laemmli Sample Buffer, then sonicated (3x30s) and boiled for 5min.

### Testis extracts

Sv129/B6 mice were euthanized at the indicated ages and testis protein extracts were prepared in RIPA buffer (25mM Tris, pH 7-8; 150mM NaCl; 0.1% SDS; 0.5% sodium deoxycholate; 1% Triton X-100; the protease inhibitors leupeptin, aprotinin and pepstatin at a final concentration of 10 µg/mL).

### Example 2: Generation of 1c chromosmes in mammalian cells by stabilization of geminin

### INTRODUCTION

The tight coordination between DNA replication and cell division allows the replication of the whole genome before each cell division, thus ensuring its accurate transmission.

This is achieved through the ordered succession of the different cell cycle phases that is regulated by several checkpoints to prevent mitotic entry during genome replication or in the case of DNA damage. DNA damage is sensed by the DNA damage checkpoint that relies on the kinases ATM and ATR to activate CHK1 or CHK2. This results in the arrest of cell cycle progression through inhibition of cyclin-dependent kinases (CDKs) and p53 stabilization that promotes transcription of cell cycle inhibitors, such as p21 and p27 (reviewed in) (Zhou and Elledge, 2000). Proper coupling of M phase to S phase is also ensured by a G2-M checkpoint that prevents premature cell entry into mitosis until DNA replication is completed (Eykelenboom et *al.,* 2013; Sorensen et *al.,* 2003; Taylor et *al.,* 1999; Zachos et *al.,* 2005; Zuazua-Villar et *al.,* 2014). More recently, the concept of a licensing checkpoint during G1 has emerged (reviewed in Ge and Blow, 2009; Hills and Diffley, 2014; Mclntosh and Blow, 2012). The assembly of a prereplication complex (pre-RC) on origins of replication (DNA replication origin licensing) occurs in late mitosis-early G1 and is the first step towards DNA replication. During pre-RC assembly, first Origin Recognition Complex (ORC) binds to replication origins. This is followed by CDC6 and CDT1 recruitment, thus enabling the loading of the replicative MCM2-7 helicase (reviewed in Fragkos et *al.,* 2015). Upon S phase entry, CDKs activate replicative complexes, while inhibiting further licensing, thus ensuring one single round of DNA replication per cell cycle. CDT1 is one of the most regulated pre-RC components. Upon replication fork activation, chromatin-bound CDT1 is degraded and CDT1 binding to chromatin is prevented by geminin, an APC/C target that is actively degraded in G1 and only expressed from S phase to mitosis. This contributes to prevent DNA replication re-initiation during S phase by restricting licensing to G1 (reviewed in Fragkos et *al.,* 2015; Machida et *al.,* 2005a). During the S phase, cells delay the activation of mitotic cyclins/CDKs until DNA replication has been completed (Eykelenboom et *al.,* 2013).

The licensing checkpoint concept arose from experiments showing that in primarymammalian cells, inhibition of the licensing reaction induces the p53-p21 axis and blocks cell cycle progression in a G1-like state in somatic cells, whereas it triggers the death of cancer cells (Lau and Jiang, 2006; Nevis et *al.,* 2009; Shreeram et *al.,* 2002, reviewed in McIntosh and Blow, 2012). This led to the hypothesis that this postulated checkpoint ensures the licensing of the correct number of origins in G1, ready to be activated before S phase onset (Lau and Jiang, 2006; Lau et *al.,* 2006; Liu et *al.,* 2009; Lunn et *al.,* 2010; Machida et *al.,* 2005b; Nevis et *al.,* 2009; Shreeram et *al.,* 2002; Teer et *al.,* 2006; Yoshida et *al.,* 2004). The licensing checkpoint, like several other checkpoints, seems to be absent also during early development, (reviewed in Kermi et *al.,* 2017). As observed in cancer cells, inhibition of DNA licensing during early development in Xenopus laevis or Drosophila does not lead to cell cycle arrest, but rather to death of cells that enter mitosis with an incompletely replicated genome (McCleland et *al.,* 2009; McGarry and Kirschner, 1998; Whittaker et *al.,* 2000). Furthermore, in haploid yeast cells, a partial DNA licensing block causes abortive replication and DNA damage checkpoint induction, whereas complete inhibition of DNA licensing in haploid yeast cells that carry a null allele of CDT1 or CDC6 leads to the occurrence of reductive mitoses and the generation of daughter cells with a reduced number of chromosomes (less than 1 C) (Hofmann and Beach, 1994; Kelly et *al.,* 1993; Piatti et *al.,* 1995). The absence of licensing checkpoint in yeast has been however proposed to be due to the lack p53 and geminin in yeast cells (Hills and Diffley, 2014; Machida et *al.,* 2005a; Nevis et *al.,* 2009).

Here, the inventors show that, in mammalian somatic cells, complete inhibition of DNA licensing obtained by inducible expression of a non-degradable geminin variant is not sensed by any checkpoint. Consequently, unlicensed cells skip the S phase and enter mitosis despite the absence of DNA replication. Most of these cells cannot complete cell division and subsequently stop proliferating and remain in a senescent G1 state. However, some can successfully terminate cell division, thus producing daughter cells that contain only half of the normal diploid complement of chromosomes (1C). Importantly, these 1C cells can re-enter into the cell cycle upon restoration of the licensing reaction by CDT1 overexpression. These observations might pave the way to new approaches to generate haploid cells from a wide range of somatic cells.

### RESULTS

### ΔDboxGeminin expression stops cell proliferation of normal cells, but does not induce checkpoints

To study the molecular mechanisms that trigger the licensing checkpoint in normal mammalian cells, the inventors generated different stable cell lines where expression of a geminin mutant lacking the Destruction box (ΔDboxGeminin) can be induced by doxycycline (DOX) **(****Figure 1****).** ΔDboxGeminin cannot be degraded and therefore, its expression is stabilized in G1 phase **(****Figure 2****),** while endogenous geminin is normally degraded upon APC/C action (McGarry and Kirschner, 1998). Upon DOX addition, ΔDboxGeminin expression was induced in 82% of cells **(****Figure 3****).**

As previously reported (Shreeram et *al.,* 2002; Yoshida et *al.,* 2004), ΔDboxGeminin expression led to massive cell death in different cancer cell lines, such as HCT116, T98G, HeLa and HEK 293 cells, as indicated by the cell number decrease, the sub-G1 population observed by cytometry and the disappearance of cells 72 hours after induction (+DOX), as observed by microscopy **(****Figure 4** and **Figures 5** **and** **6****).** Conversely and as previously observed, ΔDboxGeminin expression in untransformed cells, such as 3T3 fibroblasts and adult tail tip fibroblasts (TTFs), did not induce cell death, but blocked cell proliferation in a G1-like phase, as suggested by the accumulation of cells with 2C DNA content (2C cells) detected by cytometry **(****Figure 4** and **Figure 5****),** (Shreeram et *al.,* 2002; Yoshida et *al.,* 2004).

To understand the earliest mechanisms leading to the ΔDboxGeminin-triggered cell cycle arrest in normal cells, the inventors induced or not (+/- DOX) ΔDboxGeminin expression in synchronized 3T3 cells released from quiescence **(****Figure 7****).** Western blot analyses confirmed that ΔDboxGeminin induction in such cells prevented CDT1 binding to chromatin in G1, but did not affect its cellular abundance **(****Figures 8A and 8B****).** Analysis of chromatin fractions revealed that ΔDboxGeminin expression did not affect chromatin binding of pre-RC components that act upstream of CDT1, such as CDC6. Conversely, it dramatically reduced the chromatin association of replication factors downstream of CDT1, such as MCM2 and MCM4, thus confirming that the licensing step was inhibited by ΔDboxGeminin expression **(****Figures 8A and 8B****).** FACS analyses **(****Figure 7****)** confirmed that progression through S phase was inhibited as a consequence of licensing inhibition by ΔDboxGeminin (24% of BrdU+ cells vs 74% in non-induced cells, 14 hours after release). Importantly, 17 hours after release, 58% of ΔDboxGeminin-expressing cells were still in a G1-like state (2C DNA content and no BrdU incorporation) compared with 27% of control cells **(****Figure 7****).** The 20% of replicating cells observed upon DOX addition can be explained by the presence of a small fraction of cells in which DOX did not induce ΔDboxGeminin expression, as confirmed by FACS analyses **(****Figure 3****).** Interestingly, at later time points a defined sub-diploid cell population appeared specifically in ΔDboxGeminin-expressing cells (arrows in **Figure 7****,** see below).

It has been reported that inhibition of DNA replication licensing leads to cell cycle arrest that relies on p53 and p21 induction (Shreeram et *al.,* 2002; Yoshida et *al.,* 2004). However, despite the efficient inhibition of licensing and replication upon

ΔDboxGeminin expression, the inventors could not detect induction of the licensing checkpoint, p53 phosphorylation and p21 upregulation, at least during the first cell cycle after DOX addition (**Figure 9****,** until T0+13h). Induction of p53 upon UV-induced DNA damage confirmed the integrity of the p53 pathway in these cells **(****Figure 9****).** Furthermore, the inventors did not detect any induction of the intra-S phase checkpoint, of the G2-M checkpoint (induction of CHK1 or CHK2) or of the DNA damage checkpoint (phosphorylation of RPA32, H2AX and DNA-PK) in unlicensed cells during the first cell cycle after DOX addition **(****Figure 9****).** However, later on, when control cells (no DOX) had already passed through mitosis and started the next cell cycle (**Figure 7** T0+17h), ΔDboxGeminin-expressing cells begun to show p21 upregulation, p53 phosphorylation and expression of DNA damage markers (phosphorylated RPA32 and gH2AX detectable by western blot at T0+24h) **(****Figure 9****).** Altogether, these data show that in mammalian somatic cells, ΔDboxGeminin expression efficiently blocks origin licensing and inhibits DNA replication, without detectable checkpoint induction during the first cell cycle after DOX addition.

### Mitotic progression in the absence of DNA replication licensing in somatic cells

In line with the absence of licensing checkpoint induction, cell cycle was not blocked in ΔDboxGeminin-expressing cells. Markers of cell cycle entry showed similar kinetics in ΔDboxGeminin-expressing and control cells (no DOX) **(****Figure 10****).** Compared with T0 (release from G0), the CDK inhibitor p27 was progressively downregulated and pRB phosphorylation increased, indicating cell cycle progression. Likewise, cyclin A2, a marker of S phase entry, was normally induced, as well as markers of the G2-M phases, such as cyclin B1, PLK1 and phosphorylation of histone H3 on serine 10 (^{ser10}PH3) **(****Figure 10****).** These data confirmed the absence of checkpoint induction to block cell cycle progression after origin licensing inhibition.

The inventors then asked whether unlicensed cells could enter mitosis despite the absence of DNA replication. They synchronized 3T3 fibroblasts in G0/G1 and released them in the presence of the nucleotide analogue EdU, to monitor DNA replication during the whole cell cycle **(****Figure 11****).** In ΔDboxGeminin-expressing cells, the inventors detected mitotic cells with chromosome compaction **(****Figures 12A-H****).** Some cells, positive for ^{ser10}PH3, a mitosis marker normally expressed only in 4C cells (i.e., post-replicative cells), were devoid of any detectable EdU signal, a result never observed in control (no DOX) cells. Further immunofluorescence analyses with antibodies against a-tubulin and kinetochores (CREST) revealed that in unlicensed (EdU-negative) mitotic cells, chromosomes were aligned and bipolar spindles formed, despite the absence of replicated chromatids **(****Figures 13****).**

To further understand how mitosis could happen in cells in which licensing was inhibited, the inventors analyzed the DNA content of EdU-negative mitotic cells (as visualized by ^{ser10}PH3 expression) by flow cytometry at different time points after release from G0. In control cells (-DOX), the inventors detected ^{ser10}PH3 only in 4C cells, but never in the 2C population **(****Figures 14A-B****).** Conversely, after ΔDboxGeminin induction (+DOX), the inventors observed ^{ser10}PH3 also in 2C cells **(****Figures 14A-B****).** Importantly, the inventors obtained similar results (mitotic entry of unlicensed cells upon expression by ΔDboxGeminin) also in primary mammalian cells, such as MEFs and adult TTFs **(****Figure 15****).** Moreover, the inventors could detect ^{ser10}PH3 in ΔDboxGeminin-expressing 2C cells that were EdU-negative, as confirmed by a three-color flow cytometry approach **(****Figure 16****).** The inventors confirmed by flow cytometry the mitotic status of these 2C cells by using an antibody that targets a phosphorylated epitope present on mitotic proteins (MPM2) and an anti-cyclin B1 antibody **(****Figures 17A-B****).**

As geminin blocks initiation of DNA replication by inhibiting CDT1 (Wohlschlegel et *al.,* 2000), the inventors then asked whether CDT1 inhibition by RNA interference (shCDT1) would result in the same phenotype as upon ΔDboxGeminin expression induction **(****Figures 18-19 and Figures 20A-J****).** Indeed, the inventors could detect a ^{ser10}PH3-positive 2C cell population after CDT1 silencing and confirmed the mitotic entry of non-replicated shCDT1 cells by immunofluorescence **(****Figures 18-19 and Figures 20A-J****).**

Taken together, these data show that in somatic mammalian cells, geminin stabilization or CDT1 inhibition during the G1 phase can trigger mitotic entry of unlicensed cells, despite the inhibition of DNA replication.

### A discrete population of unlicensed cells can successfully divide

The inventors then investigated whether unlicensed cells that entered mitosis could divide. To this aim, the inventors prepared metaphase spreads of MEFs 10 hours after synchronization in G0/G1 and release in the presence of EdU (with or without DOX). Control cells (-ΔDboxGem) had 40 pairs of chromosomes with the two replicated (EdU-positive) sister chromatids attached by the kinetochore **(****Figure 21****).** Conversely, ΔDboxGeminin expressing cells were EdU-negative and had 40 single-chromatid chromosomes. To determine whether unlicensed cells could go through mitosis and also divide, the inventors synchronized in G0/G1 3T3 cells that transiently express GFP-tagged histone 2B (GFP-H2B), and then released them in the presence of DOX and EdU, to detect DNA synthesis **(****Figures 22****).** The inventors used time lapse microscopy to monitor mitotic progression **(****Figures 23** **and** **24****)** and then, the inventors fixed cells to check for the presence/absence of DNA replication before mitosis. Figure 4D shows an example of dividing cell without detectable DNA synthesis. Overall, 24% of unlicensed cells (EdUnegative, white arrows in **Figure 24**) that entered mitosis could successfully divide (Supplementary movies), whereas the other EdU-negative cells (76%) could not. This latter population resulted in binucleated cells (cytokinesis failure, **Figure 24**) or in cells with a single nucleus (mitotic slippage, **Figure 24**).

In summary, although most unlicensed cells cannot complete cell division, about onequarter can produce two daughter cells. The inventors then assessed the fate of these two cell populations.

### Mitotic division failure in unlicensed mitotic cells leads to a long-term irreversible G1 arrest with features of senescence

To investigate the fate of unlicensed 3T3 cells that entered mitosis but did not complete cell division, the inventors analyzed them by immunofluorescence at different time points after release from G0 in the presence or not of DOX. Quantification of the percentage of ^{ser10}PH3-positive cells showed that the first wave of mitoses started 14h post-release with a peak after 17h **(****Figure 25****).** Just after the beginning of mitotic entry, ΔDboxGeminin-expressing cells exhibited a strong increase of anaphase bridges and micronuclei **(****Figure 25** and **26****),** confirming the mitosis completion defects in unlicensed mitotic cells observed by time lapse microscopy **(****Figure 24****).** In addition, ΔDboxGeminin-positive cells showed unusually large and weak foci of phosphorylated H2AX (gH2AX) that decorated nucleoli after the first wave of mitoses **(****Figures 27A-H** and **Figures 28AH**). These persistent nucleolar are evocative to those previously observed in old hematopoietic cells undergoing senescence (Flach et *al.,* 2014). Indeed, cells with nucleolar gH2AX foci were always EdU-negative **(****Figures 27A-H****),** suggesting that they have exited the cell cycle. In contrast to the inventors observations during the first cell cycle after ΔDboxGeminin induction (i.e., T0+13h), the DNA damage checkpoint activators p53 and p21 were induced at these later "post-mitotic" time points (T0+48h) (compare **Figures 28A-H** with Figure 5E **Figure 29**), as previously reported (Shreeram et *al.,* 2002). Moreover, 14 days after ΔDboxGeminin induction, most cells were still alive, but had entered a state of premature senescence, as revealed by b-galactosidase staining **(****Figure 30****).**

The inventors' data indicate that the population of unlicensed mitotic cells that failed cell division is subsequently blocked in G1. This is associated with atypical nucleolar DNA damage, induction of the DNA damage checkpoint and features of senescence that only appear after the first mitotic wave. Therefore, induction of p53 and p21 in unlicensed cells is not primarily triggered by lack of licensed origins, but rather by mitotic failure-induced DNA damage, as observed after abortive completion of normal (4C DNA content, postreplicative) mitoses (Hayashi and Karlseder, 2013; Panopoulos et *al.,* 2014).

### Division of unlicensed cells leads to a cell population with 1C DNA content.

The fraction of unlicensed cells (24%) that managed to divide produced two daughter cells, despite the complete absence of DNA replication. If the unlicensed genome were equally distributed in each of the two daughter cells originating from a mitotic cell with 2C DNA content, these cells should have 1C DNA content. Indeed, the inventors flow cytometry analyses always highlighted the presence of a distinct cell population among ΔDboxGeminin-expressing cells with 1C DNA content that appeared after the first cell cycle (arrows in **Figure 4** and **Figure 31**). The inventors confirmed that they were daughter cells (i.e., the result of mitosis) because inhibition of mitotic entry by incubation with the CDK1 inhibitor RO-3306 totally prevented their appearance **(****Figure 31****).** Moreover, the inventors could not detect cleaved caspase 3 (a marker of apoptosis) in these daughter cells, in contrast to control UV-irradiated cells **(****Figure 32****).** Moreover, after UV exposure, cell cycle progression of unlicensed cells was inhibited by the DNA damage checkpoint (as indicated by p53 phosphorylation; **Figure 33**) and this prevented the appearance of 1C cells (**Figure 31****).** Noticeably, the sub-G1 population of ΔDboxGeminin-expressing cells (with 1C DNA content) showed a sharp profile, as opposed to the classic spreadout profile of dying sub-G1 cells after UV irradiation **(****Figure 31****).**

To determine the number of chromosomes in this cell population, the inventors counted the number of kinetochores stained by the CREST polyclonal antibody in mouse embryonic stem cells that exhibit a more stable diploid karyotype than 3T3 fibroblasts and MEFs. In the inventors conditions, the inventors found an average of 31.5 dots per nucleus in control diploid cells (theoretically, 40 dots, one per chromosome, are expected) **(****Figures 34A-D** and **Figure 35****).** This discrepancy could be explained by the close proximity of some kinetochores in interphase. In ΔDboxGeminin-expressing cells (but never in control cells), 7% of nuclei had up to 20 dots (mean: 15.7 dots), a proportion similar to the fraction of 1C cells detected by flow cytometry (see **Figure 31** and **Figure 35**). The average number of 15.7 dots per nucleus corresponds, when considering the similar bias of detection than in control cells, to 19.6 kinetochores, a number close to the theoretical 20 dots per nucleus of 1C cells.

### The 1C cell population can re-enter S phase

Finally, the inventors investigated whether 1C cells could re-enter the cell cycle after stopping ΔDboxGeminin action. First, the inventors removed DOX from the medium 24 hours after induction, to stop ΔDboxGeminin expression (T1, **Figure 36****;** control cells in which ΔDboxGeminin was not induced are shown in **Figure 37**). Then, the inventors transfected these cells with a CDT1-encoding plasmid (or empty vector; control) to counteract the effect of the remaining ΔDboxGeminin. Analysis by flow cytometry of BrdU incorporation by 1C cells showed that at T1, most (98.5%) 1C cells were not replicating (**Figure 36****;** BrdU-negative: blue dashed square, left panel). However, at T2 (24h after CDT1 transfection), 15.3% of 1C cells were BrdU-positive (**Figure 36****,** BrdU 24h, right panels) compared with 4.9% of 1C cells transfected with empty vector.

To rule out the possibility that these BrdU-positive 1C cells corresponded to diploid cells that replicated and died during the 24 hours preceding cell harvesting, the inventors performed a short BrdU pulse (15 minutes) just before cell collection at T2. In these conditions, 8.3% of 1C cells transfected with CDT1 were still actively replicating compared with 2.7% of cells transfected with empty vector (Figure 36, BrdU pulse, T2+CDT1 and T2+empty vector). These results show that 1C cells can re-enter S phase after re-establishment of licensing by expression of CDT1.

Taken together, these data show that in mammalian cells, reductive division of unlicensed mitotic cells can lead to the appearance of 1C daughter cells that can survive and re-enter into the cell cycle if the licensing reaction is rescued.

### DISCUSSION

### Revisiting the licensing checkpoint

The existence of a mammalian somatic cell-specific licensing checkpoint has been suggested by the G1-like block observed in normal cells in which licensing was inhibited by siRNA-based approaches (Lau et al., 2009; Liu et al., 2009; Lunn et al., 2010; Machida et *al.,* 2005b; Nevis et *al.,* 2009; Teer et *al.,* 2006). As most of the pre- RC components are highly stable in G1 as soon as they are bound to origins, it is challenging to completely stop the licensing reaction in a single cell cycle by interfering with their expression during pre-RC formation. This aspect is crucial because previous observations made in yeast show that a licensed origin will fire regardless of the number of licensed origins (Piatti et *al.,* 1995; Shimada et *al.,* 2002). Therefore, only a complete pre-RC inhibition can prevent the activation of the intra S-phase checkpoint. Here, the inventors took advantage of different mammalian cell lines that express an inducible and not degradable geminin to completely block replication licensing in a single cell cycle. The inventors data show that in mammalian somatic cells, complete inhibition of CDT1 action and therefore of licensing in G1 is not sensed by any cell cycle checkpoint during the first cell cycle, leading to the entry of unlicensed cells into mitosis. This suggests that in mammalian somatic cells, a licensing checkpoint is not activated when CDT1 action is fully prevented. As the DNA content of unlicensed cells remains unchanged despite their cell cycle progression, these cells cannot be distinguished from a typical G0/G1 population by quantifying the DNA content, and could easily be missed when only measuring the DNA content using a single flow cytometry approach.

### G1-like cell cycle arrest upon licensing inhibition

As illustrated in **Figure 39****,** unlicensed cells may have two different fates which depend on the success of cell division. The cell cycle arrest and senescence resulting from mitotic failure of the majority of non-replicated cells are reminiscent of the senescence observed in normal cells after interference with mitotic progression (Andreassen et *al.,* 2001; Fong et *al.,* 2016; Meitinger et *al.,* 2016; Panopoulos et *al.,* 2014). Mitotic delay or failure can induce H2AX phosphorylation and the DNA damage response in normal diploid cells that are undergoing tetraploidization or mitotic perturbations (Janssen et *al.,* 2011, Hayashi and Karlseder, 2013). Here, the inventors show that mitotic entry is preceding p53/p21 activation and H2AX phosphorylation in unlicensed cells. Therefore, DNA damage (and the subsequent cell cycle block in G1) appears to be the consequence of mitotic failure of unlicensed cells, rather than of the absence of licensing or DNA replication. This suggests that mitotic failure induces senescence regardless of any replicative process and explain why cancer cells that are more susceptible to cell death upon mitotic perturbations than normal cells, massively died upon licensing inhibition (Janssen et *al.,* 2009; Mc Gee, 2015). Interestingly, the inventors found that G1-like arrested cells display nucleolar gH2AX foci, similar to what observed in hematopoietic cells undergoing senescence (Alvarez et *al.,* 2015; Flach et *al.,* 2014). Further studies may clarify the potential role of these gH2AX nucleolar foci in senescence induction.

### Licensing inhibition to generate haploid mammalian cells

The inventors' data show that some unlicensed cells can establish a mitotic spindle and divide despite the absence of DNA replication. This is in line with the observation that the spindle assembly checkpoint can be satisfied even in the absence of DNA replication in cancer cells after DNA replication and checkpoint inhibition (Brinkley et *al.,* 1988; O'Connell et *al.,* 2009) and in immortalized Drosophila embryonic S2 cells (Drpic et *al.,* 2015). Importantly, some unlicensed mammalian somatic cells can eventually complete mitosis, giving raise to two daughter cells. It is also worth noting that geminin expression is strongly upregulated during testis gametogenesis, particularly when meiosis is induced **(****Figure 38****).** Meiosis is characterized by the lack of DNA replication between the first and the second successive meiotic divisions, and meiosis onset in mouse germ cells is accompanied by a strong upregulation of geminin between meiosis I and meiosis II, (Ma et *al.,* 2016). Therefore, a stable geminin induced "reductive somatic mitosis" could mimic some features of the first reductive mitosis in meiotic cells.

The 1C daughter cells derived from unlicensed somatic mammalian cells are viable and show a relative homogenous DNA content. Some of them contain up to 20 chromosomes, suggesting that they could be fully haploid. Moreover, after reestablishment of the licensing reaction, these cells can enter the cell cycle and even replicate DNA. The inventors' demnotrate a new experimental approach to generate haploid cells from a wide range of somatic mammalian cells that could become a powerful tool for the development of genetic and drug screens (Carette et *al.,* 2009; Sagi et *al.,* 2016).

### REFERENCE

Alvarez, S., Diaz, M., Flach, J., Rodriguez-Acebes, S., Lopez-Contreras, A.J., Martinez, D., Canamero, M., Fernandez-Capetillo, O., Isern, J., Passegue, E., et al. (2015). Replication stress caused by low MCM expression limits fetal erythropoiesis and hematopoietic stem cell functionality. Nat Commun 6, 8548.
Andreassen, P.R., Lohez, O.D., Lacroix, F.B., and Margolis, R.L. (2001). Tetraploid state induces p53-dependent arrest of nontransformed mammalian cells in G1. Mol Biol Cell 12, 1315-1328.
Brinkley, B.R., Zinkowski, R.P., Mollon, W.L., Davis, F.M., Pisegna, M.A., Pershouse, M., and Rao, P.N. (1988). Movement and segregation of kinetochores experimentally detached from mammalian chromosomes. Nature 336, 251-254.
Carette, J.E., Guimaraes, C.P., Varadarajan, M., Park, A.S., Wuethrich, I., Godarova, A., Kotecki, M., Cochran, B.H., Spooner, E., Ploegh, H.L., et al. (2009). Haploid genetic screens in human cells identify host factors used by pathogens. Science 326, 1231-1235.
Drpic, D., Pereira, A.J., Barisic, M., Maresca, T.J., and Maiato, H. (2015). Polar Ejection Forces Promote the Conversion from Lateral to End-on Kinetochore-Microtubule Attachments on Mono-oriented Chromosomes. Cell Rep 13, 460-469.
Eykelenboom, J.K., Harte, E.C., Canavan, L., Pastor-Peidro, A., Calvo-Asensio, I., Llorens-Agost, M., and Lowndes, N.F. (2013). ATR activates the S-M checkpoint during unperturbed growth to ensure sufficient replication prior to mitotic onset. Cell Rep 5, 1095-1107.
Flach, J., Bakker, S.T., Mohrin, M., Conroy, P.C., Pietras, E.M., Reynaud, D., Alvarez, S., Diolaiti, M.E., Ugarte, F., Forsberg, E.C., et al. (2014). Replication stress is a potent driver of functional decline in ageing haematopoietic stem cells. Nature 512, 198-202.
Fong, C.S., Mazo, G., Das, T., Goodman, J., Kim, M., O'Rourke, B.P., Izquierdo, D., and Tsou, M.F. (2016). 53BP1 and USP28 mediate p53-dependent cell cycle arrest in response to centrosome loss and prolonged mitosis. eLife 5.
Fragkos, M., Ganier, O., Coulombe, P., and Mechali, M. (2015). DNA replication origin activation in space and time. Nat Rev Mol Cell Biol 16, 360-374.
Ge, X.Q., and Blow, J.J. (2009). The licensing checkpoint opens up. Cell Cycle 8, 2320-2322.
Hayashi, M.T., and Karlseder, J. (2013). DNA damage associated with mitosis and cytokinesis failure. Oncogene 32, 4593-4601.
Hills, S.A., and Diffley, J.F. (2014). DNA replication and oncogene-induced replicative stress. Current biology : CB 24, R435-444.
Hofmann, J.F., and Beach, D. (1994). cdt1 is an essential target of the Cdc10/Sct1 transcription factor: requirement for DNA replication and inhibition of mitosis. Embo J 13, 425-434.
Janssen, A., Kops, G.J., and Medema, R.H. (2009). Elevating the frequency of chromosome mis-segregation as a strategy to kill tumor cells. Proc Natl Acad Sci U S A 106, 19108-19113.
Janssen, A., van der Burg, M., Szuhai, K., Kops, G.J., and Medema, R.H. (2011). Chromosome segregation errors as a cause of DNA damage and structural chromosome aberrations. Science 333, 1895-1898.
Kelly, T.J., Martin, G.S., Forsburg, S.L., Stephen, R.J., Russo, A., and Nurse, P. (1993). The fission yeast cdc18+ gene product couples S phase to START and mitosis. Cell 74, 371-382.
Kermi, C., Lo Furno, E., and Maiorano, D. (2017). Regulation of DNA Replication in Early Embryonic Cleavages. Genes 8.
Lau, E., Chiang, G.G., Abraham, R.T., and Jiang, W. (2009). Divergent S phase checkpoint activation arising from prereplicative complex deficiency controls cell survival. Mol Biol Cell 20, 3953-3964.
Lau, E., and Jiang, W. (2006). Is there a pre-RC checkpoint that cancer cells lack? Cell Cycle 5, 1602-1606.
Lau, E., Zhu, C., Abraham, R.T., and Jiang, W. (2006). The functional role of Cdc6 in S-G2/M in mammalian cells. EMBO Rep 7, 425-430.
Liu, P., Slater, D.M., Lenburg, M., Nevis, K., Cook, J.G., and Vaziri, C. (2009). Replication licensing promotes cyclin D1 expression and G1 progression in untransformed human cells. Cell Cycle 8, 125-136.
Lunn, C.L., Chrivia, J.C., and Baldassare, J.J. (2010). Activation of Cdk2/Cyclin E complexes is dependent on the origin of replication licensing factor Cdc6 in mammalian cells. Cell Cycle 9, 4533-4541.
Ma, X.S., Lin, F., Wang, Z.W., Hu, M.W., Huang, L., Meng, T.G., Jiang, Z.Z., Schatten, H., Wang, Z.B., and Sun, Q.Y. (2016). Geminin deletion in mouse oocytes results in impaired embryo development and reduced fertility. Mol Biol Cell 27, 768-775.
Machida, Y.J., Hamlin, J.L., and Dutta, A. (2005a). Right place, right time, and only once: replication initiation in metazoans. Cell 123, 13-24.
Machida, Y.J., Teer, J.K., and Dutta, A. (2005b). Acute reduction of an origin recognition complex (ORC) subunit in human cells reveals a requirement of ORC for Cdk2 activation. J Biol Chem 280, 27624-27630.
Mc Gee, M.M. (2015). Targeting the Mitotic Catastrophe Signaling Pathway in Cancer. Mediators Inflamm 2015, 146282.
McCleland, M.L., Shermoen, A.W., and O'Farrell, P.H. (2009). DNA replication times the cell cycle and contributes to the mid-blastula transition in Drosophila embryos. J Cell Biol 187, 7-14.
McGarry, T.J., and Kirschner, M.W. (1998). Geminin, an inhibitor of DNA replication, is degraded during mitosis. Cell 93, 1043-1053.
McIntosh, D., and Blow, J.J. (2012). Dormant origins, the licensing checkpoint, and the response to replicative stresses. Cold Spring Harb Perspect Biol 4.
Meitinger, F., Anzola, J.V., Kaulich, M., Richardson, A., Stender, J.D., Benner, C., Glass, C.K., Dowdy, S.F., Desai, A., Shiau, A.K., et al. (2016). 53BP1 and USP28 mediate p53 activation and G1 arrest after centrosome loss or extended mitotic duration. J Cell Biol 214, 155-166.
Nevis, K.R., Cordeiro-Stone, M., and Cook, J.G. (2009). Origin licensing and p53 status regulate Cdk2 activity during G(1). Cell Cycle 8, 1952-1963.
O'Connell, C.B., Loncarek, J., Kalab, P., and Khodjakov, A. (2009). Relative contributions of chromatin and kinetochores to mitotic spindle assembly. J Cell Biol 187, 43-51.
Panopoulos, A., Pacios-Bras, C., Choi, J., Yenjerla, M., Sussman, M.A., Fotedar, R., and Margolis, R.L. (2014). Failure of cell cleavage induces senescence in tetraploid primary cells. Mol Biol Cell 25, 3105-3118.
Piatti, S., Lengauer, C., and Nasmyth, K. (1995). Cdc6 is an unstable protein whose de novo synthesis in G1 is important for the onset of S phase and for preventing a 'reductional' anaphase in the budding yeast Saccharomyces cerevisiae. EMBO J 14, 3788-3799.
Sagi, I., Chia, G., Golan-Lev, T., Peretz, M., Weissbein, U., Sui, L., Sauer, M.V., Yanuka, O., Egli, D., and Benvenisty, N. (2016). Derivation and differentiation of haploid human embryonic stem cells. Nature 532, 107-111.
Shimada, K., Pasero, P., and Gasser, S.M. (2002). ORC and the intra-S-phase checkpoint: a threshold regulates Rad53p activation in S phase. Genes Dev 16, 3236-3252.
Shreeram, S., Sparks, A., Lane, D.P., and Blow, J.J. (2002). Cell type-specific responses of human cells to inhibition of replication licensing. Oncogene 21, 6624-6632.
Sorensen, C.S., Syljuasen, R.G., Falck, J., Schroeder, T., Ronnstrand, L., Khanna, K.K., Zhou, B.B., Bartek, J., and Lukas, J. (2003). Chk1 regulates the S phase checkpoint by coupling the physiological turnover and ionizing radiation-induced accelerated proteolysis of Cdc25A. Cancer Cell 3, 247-258.
Taylor, W.R., Agarwal, M.L., Agarwal, A., Stacey, D.W., and Stark, G.R. (1999). p53 inhibits entry into mitosis when DNA synthesis is blocked. Oncogene 18, 283-295.
Teer, J.K., Machida, Y.J., Labit, H., Novac, O., Hyrien, O., Marheineke, K., Zannis-Hadjopoulos, M., and Dutta, A. (2006). Proliferating human cells hypomorphic for origin recognition complex 2 and pre-replicative complex formation have a defect in p53 activation and Cdk2 kinase activation. J Biol Chem 281, 6253-6260.
Whittaker, A.J., Royzman, I., and Orr-Weaver, T.L. (2000). Drosophila double parked: a conserved, essential replication protein that colocalizes with the origin recognition complex and links DNA replication with mitosis and the down-regulation of S phase transcripts. Genes Dev 14, 1765-1776.
Wohlschlegel, J.A., Dwyer, B.T., Dhar, S.K., Cvetic, C., Walter, J.C., and Dutta, A. (2000). Inhibition of eukaryotic DNA replication by geminin binding to Cdt1. Science 290, 2309-2312.
Yoshida, K., Oyaizu, N., Dutta, A., and Inoue, I. (2004). The destruction box of human Geminin is critical for proliferation and tumor growth in human colon cancer cells. Oncogene 23, 58-70.
Zachos, G., Rainey, M.D., and Gillespie, D.A. (2005). Chk1-dependent S-M checkpoint delay in vertebrate cells is linked to maintenance of viable replication structures. Mol Cell Biol 25, 563-574.
Zhou, B.B., and Elledge, S.J. (2000). The DNA damage response: putting checkpoints in perspective. Nature 408, 433-439.
Zuazua-Villar, P., Rodriguez, R., Gagou, M.E., Eyers, P.A., and Meuth, M. (2014). DNA replication stress in CHK1-depleted tumour cells triggers premature (S-phase) mitosis through inappropriate activation of Aurora kinase B. Cell death & disease 5, e1253.

The invention is not limited to the embodiments described above and other embodiments will clearly appear from the specification to the skilled person.

## Claims

1. Use of an inhibitor of the activity or stability of the pre-replication complex, also called pre-RC, for the *in vitro* preparation of haploid eukaryotic somatic cells from eukaryotic somatic diploid cells, said inhibitor completely inhibiting the activity or stability of said pre-RC.

2. Use according to claim 1, wherein said inhibitor is an inhibitor of the activity or expression of the chromatin licensing and DNA replication factor 1 protein, also called Cdt 1.

3. Use according to claim 1 or 2, wherein said inhibitor is a non proteasome-degradable form of the Geminin protein.

4. Use according to claim 3, wherein said non proteasome-degradable form of the Geminin protein lacks the following sequence: **RxTLKxzQx.** (SEQ ID NO : 2), wherein x represents any amino acid and z represent a leucine (L) or a valine (V).

5. Use according to claim 2, wherein said inhibitor is a small interfering molecule inhibiting the expression of said Cdt 1 protein.

6. Use according to anyone of claims, wherein said haploid eukaryotic somatic cells are able to carry out mitosis.

7. Method for producing *in vitro* eukaryotic somatic haploid cells from eukaryotic somatic diploid cells, said method comprising a step of contacting eukaryotic somatic diploid cells with an inhibitor of the activity or stability of pre-RC, said inhibitor completely inhibiting the activity or stability of said pre-RC.

8. Method according to claim 7, wherein said inhibitor is either an inhibitor of the activity or expression of the Cdt1 protein, or said inhibitor is a small interfering molecule inhibiting the expression of said Cdt 1 protein.

9. Method according to claim 7, wherein said inhibitor is a non degradable form of the Geminin protein, in particular a Geminin protein lacks the following sequence: : **RxTLKxzQx.** (SEQ ID NO : 2), wherein x represents any amino acid and z represent a leucine (L) or a valine (V).

10. An isolated eukaryotic somatic haploid cell liable to be obtained by the method according to anyone of claims 7 to 9.

11. An isolated eukaryotic somatic haploid cell provided that said eukaryotic somatic haploid cell is not an embryonic haploid stem cells, said somatic cell being **characterized in that** it harbors differentiation epigenetic marks.

12. Nucleus of an isolated eukaryotic somatic haploid cell as defined in claim 10 or in claim 11.

13. A method for carrying out an *in vitro* fecundation comprising a step of introducing into a female germinal egg a nucleus of an isolated eukaryotic somatic haploid cell as defined in claim 12.

14. A method for the *in vitro* production of homozygote cells for at least a determined locus, said method comprising a step of genetically modifying at least one locus of a eukaryotic somatic haploid cell as defined in claim 10 or in claim 11, a step of inducing DNA replication of said eukaryotic somatic haploid cell and a step of inhibiting cell division.

15. A nucleic acid molecule coding for a mutated geminin protein, said protein containing a deletion of the sequence : **RxTLKxzQx.** (SEQ ID NO : 2), wherein x represents any amino acid and z represent a leucine (L) or a valine (V), said nucleic acid sequence being possibly contained in a vector allowing the expression of said nucleic acid sequence further to a conditional activation.


SEQUENCE LISTING

<110>  Centre national de la recherche scientifique

<120>  Method for obtaining haploid cells

<130>  BR84691

<160>  12

<170>  PatentIn version 3.5

<210>  1
<211>  9
<212>  PRT
<213>  Artificial sequence

<220>
<223>  murine deletion box

<400>  1

Arg Arg Thr Leu Lys Met Ile Gln Pro
1               5


<210>  2
<211>  9
<212>  PRT
<213>  Artificial Sequence

<220>
<223>  consensus degradation box


<220>
<221>  misc_feature
<222>  (2)..(2)
<223>  Xaa can be any naturally occurring amino acid

<220>
<221>  misc_feature
<222>  (6)..(6)
<223>  Xaa can be any naturally occurring amino acid

<220>
<221>  MISC_FEATURE
<222>  (7)..(7)
<223>  Xaa = L or V

<220>
<221>  MISC_FEATURE
<222>  (7)..(7)
<223>  Xaa = Leu  or Val

<220>
<221>  misc_feature
<222>  (9)..(9)
<223>  Xaa can be any naturally occurring amino acid

<400>  2

Arg Xaa Thr Leu Lys Xaa Xaa Gln Xaa

1                   5


<210>  3
<211>  209
<212>  PRT
<213>  consensus deletion box

<400>  3

<210>  4
<211>  206
<212>  PRT
<213>  Mus musculus

<400>  4

Met Asn Leu Ser Met Lys Gln Lys Gln Glu Gly Ala Gln Glu Asn Val
1               5                   10                  15

Lys Asn Ser Pro Val Pro Arg Arg Thr Leu Lys Met Ile Gln Pro Ser
            20                  25                  30

Ala Asp Gly Ser Leu Val Gly Arg Glu Asn Glu Leu Pro Lys Gly Leu
        35                  40                  45

Phe Lys Arg Lys Leu Trp Asp Asp Gln Leu Ala Ser Gln Thr Ser Ser
    50                  55                  60

Cys Gly Pro Glu Ala Asn Glu Asn Lys Asp Val Gly Asp Leu Thr Gln
65                  70                  75                  80

Glu Ala Phe Asp Leu Ile Ser Lys Glu Asn Pro Ser Ser Gln Tyr Trp
                85                  90                  95

Lys Glu Val Ala Glu Gln Arg Arg Lys Ala Leu Tyr Glu Ala Leu Lys
            100                 105                 110

Glu Asn Glu Lys Leu His Lys Glu Ile Glu Gln Lys Asp Ser Glu Ile
        115                 120                 125

Ala Arg Leu Arg Lys Glu Asn Lys Asp Leu Ala Glu Val Ala Glu His
    130                 135                 140

Val Gln Tyr Met Ala Glu Val Ile Glu Arg Leu Ser Asn Glu Pro Leu
145                 150                 155                 160

Asp Asn Phe Glu Ser Pro Asp Ser Gln Glu Phe Asp Ser Glu Glu Glu
                165                 170                 175

Ala Val Glu Tyr Ser Glu Leu Glu Asp Ser Gly Ala Gly Thr Cys Ala
            180                 185                 190

Glu Glu Thr Val Ser Ser Ser Thr Asp Ala Arg Pro Cys Thr
        195                 200                 205

<210>  5

<211>  216
<212>  PRT
<213>  Xenopus laevis

<400>  5

Met Asn Ser Asn Met Lys Gln Arg Ser Asp Val Glu Asn Pro Ser Met
1               5                   10                  15

Ser Ile Lys Asn Tyr Ile Val Asp Lys Thr Asn Glu Ala Leu Ala Pro
            20                  25                  30

Arg Arg Thr Leu Lys Val Ile Gln Gln Ser Ala Ser Gly Cys Leu Val
        35                  40                  45

Gly Arg Thr Lys Glu Pro Ala Lys Asn Ser Thr Lys Arg Lys Leu Trp
    50                  55                  60

Asn Asp Gln Leu Thr Ser Lys Lys Ala Lys Val Glu Val Ala Val Asp
65                  70                  75                  80

Pro Glu His Gln Glu Asn Lys Asp Cys Pro Ser Glu Ala Tyr Asp Leu
                85                  90                  95

Met Val Lys Glu Thr Pro Thr Cys Leu Tyr Trp Lys Asp Val Ala Glu
            100                 105                 110

Glu Arg Arg Lys Ala Leu Tyr Glu Ala Leu Gln Glu Asn Glu Lys Leu
        115                 120                 125

His Gln Glu Ile Glu Leu Lys Asp Glu Glu Ile Ala Arg Leu Lys Gln
    130                 135                 140

Glu Asn Asp Glu Leu Met Glu Leu Ala Gly His Val Gln Tyr Met Ala
145                 150                 155                 160

Asn Met Ile Glu Arg Leu Thr Gly Asn Ala Pro Gln Ser Leu Glu Asp
                165                 170                 175

Leu Lys Asn Leu Asp Leu Glu Glu Ala Arg Phe Glu Asp Glu Ala Glu
            180                 185                 190

Ser Arg Ile Glu Asp Glu Thr Asp Met Thr Gln Pro Ser Ser Ser Asp
        195                 200                 205

Gln Asn Met Asp Lys Gln Thr Val
    210                 215

<210>  6

<211>  219
<212>  PRT
<213>  Xenopus laevis

<400>  6

Met Asn Thr Asn Lys Lys Gln Arg Leu Asp Met Glu Lys Pro Thr Met
1               5                   10                  15

Ser Ile Lys Asn Tyr Phe Val Asp Lys Thr Asn Glu Ser Leu Ala Pro
            20                  25                  30

Arg Arg Thr Leu Lys Val Ile Gln Pro Ser Ala Ser Gly Cys Leu Val
        35                  40                  45

Gly Arg Thr Lys Glu Pro Val Lys Asn Ser Thr Lys Arg Lys Leu Trp
    50                  55                  60

Asn Asp Gln Leu Thr Ser Lys Lys Ala Lys Val Glu Val Ala Val Asp
65                  70                  75                  80

Pro Glu His Arg Glu Asn Lys Asp Cys Ser Ser Glu Ala Tyr Asp Leu
                85                  90                  95

Met Val Lys Glu Thr Pro Thr Cys Leu Tyr Trp Lys Glu Val Ala Glu
            100                 105                 110

Glu Arg Arg Lys Ala Leu Tyr Glu Ala Leu Gln Glu Asn Glu Lys Leu
        115                 120                 125

His Lys Glu Ile Glu Leu Lys Asp Glu Glu Ile Ala Arg Leu Lys Gln
    130                 135                 140

Glu Asn Asp Glu Leu Met Glu Leu Ala Gly His Val Gln Tyr Met Ala
145                 150                 155                 160

Asn Met Ile Glu Arg Leu Thr Gly Asn Ala Pro Arg Ser Leu Glu Asp
                165                 170                 175

Leu Lys Asp Leu Asp Leu Glu Glu Ala Arg Phe Glu Asp Glu Ala Asp
            180                 185                 190

Met Ala Glu Ala Arg Ile Glu Asp Glu Thr Asp Met Ala Arg Pro Ser
        195                 200                 205

Asn Ser Asp Gln Asn Met Asp Ala His Thr Val
    210                 215

<210>  7

<211>  192
<212>  PRT
<213>  Xenopus laevis

<400>  7

Met Ser Ser Ser Ala Ala Arg Val Tyr Ile Gln Val Glu Thr Glu Ala
1               5                   10                  15

Glu Gln Lys Glu His Leu Lys Gln Gln Arg Gln Thr Leu Lys Pro Leu
            20                  25                  30

Gln Gly Asn Val Asn Asp Lys Glu Asn Leu Thr Gly Ser Gly Arg Ala
        35                  40                  45

Ser Ile Val Asp Gln Leu Ser Arg Leu Lys Ala Gly Val Gln Val Ala
    50                  55                  60

Pro Lys Tyr Gly Lys Arg Lys Cys Val Asp Thr Ala Pro Ala Ile Ser
65                  70                  75                  80

Thr Lys Asp Ala Asp Thr Gln Thr Asp Ala Glu Ala Val Pro Leu Gly
                85                  90                  95

Asn Ala Asp Lys Pro Ile Thr Ala Glu Asp Leu Thr Ser Thr Ala Glu
            100                 105                 110

Pro Gly Glu Asn Tyr Tyr Lys Leu Leu Ala Glu Gln Arg Arg Leu Ala
        115                 120                 125

Leu Glu Asp Ser Leu Thr Glu Asn Arg His Leu His Glu Arg Ile Glu
    130                 135                 140

Gly Leu Glu Glu Glu Met Asp Thr Met Arg Gln Glu Leu Asp Glu Ala
145                 150                 155                 160

Lys Asn Leu Val Glu Val Leu Lys Glu Ile Cys Glu Glu Asp Asn Ser
                165                 170                 175

Glu Val Glu Glu Asp Asp Thr Thr Gly Asp Glu Asp Lys Val Asn Ala
            180                 185                 190


<210>  8
<211>  192
<212>  PRT
<213>  Xenopus laevis

<400>  8

Met Ser Ser Ser Ala Ala Arg Val Tyr Ile Gln Val Glu Thr Glu Ala
1               5                   10                  15

Glu Gln Lys Glu His Leu Lys Gln Gln Arg Gln Thr Leu Lys Pro Leu
            20                  25                  30

Gln Gly Asn Val Asn Asp Lys Glu Asn Leu Thr Gly Ser Gly Arg Ala
        35                  40                  45

Ser Ile Val Asp Gln Leu Ser Arg Leu Lys Ala Gly Val Gln Val Ala
    50                  55                  60

Pro Lys Tyr Gly Lys Arg Lys Cys Val Asp Thr Ala Pro Ala Ile Ser
65                  70                  75                  80

Thr Lys Asp Ala Asp Thr Gln Thr Asp Ala Glu Ala Val Pro Leu Gly
                85                  90                  95

Asn Ala Asp Lys Pro Ile Thr Ala Glu Asp Leu Thr Ser Thr Ala Glu
            100                 105                 110

Pro Gly Glu Asn Tyr Tyr Lys Leu Leu Ala Glu Gln Arg Arg Leu Ala
        115                 120                 125

Leu Glu Asp Ser Leu Thr Glu Asn Arg His Leu His Glu Arg Ile Glu
    130                 135                 140

Gly Leu Glu Glu Glu Met Asp Thr Met Arg Gln Glu Leu Asp Glu Ala
145                 150                 155                 160

Lys Asn Leu Val Glu Val Leu Lys Glu Ile Cys Glu Glu Asp Asn Ser
                165                 170                 175

Glu Val Glu Glu Asp Asp Thr Thr Gly Asp Glu Asp Lys Val Asn Ala
            180                 185                 190

<210>  9
<211>  201
<212>  PRT
<213>  Mus musculus

<400>  9

Met Asn Leu Ser Met Lys Gln Lys Gln Glu Gly Ala Gln Glu Asn Val
1               5                   10                  15

Lys Asn Ser Pro Val Pro Ala Ala Ala Pro Ser Ala Asp Gly Ser Leu
            20                  25                  30

Val Gly Arg Glu Asn Glu Leu Pro Lys Gly Leu Phe Lys Arg Lys Leu
        35                  40                  45

Trp Asp Asp Gln Leu Ala Ser Gln Thr Ser Ser Cys Gly Pro Glu Ala
    50                  55                  60

Asn Glu Asn Lys Asp Val Gly Asp Leu Thr Gln Glu Ala Phe Asp Leu
65                  70                  75                  80

Ile Ser Lys Glu Asn Pro Ser Ser Gln Tyr Trp Lys Glu Val Ala Glu
                85                  90                  95

Gln Arg Arg Lys Ala Leu Tyr Glu Ala Leu Lys Glu Asn Glu Lys Leu
            100                 105                 110

His Lys Glu Ile Glu Gln Lys Asp Ser Glu Ile Ala Arg Leu Arg Lys
        115                 120                 125

Glu Asn Lys Asp Leu Ala Glu Val Ala Glu His Val Gln Tyr Met Ala
    130                 135                 140

Glu Val Ile Glu Arg Leu Ser Asn Glu Pro Leu Asp Asn Phe Glu Ser
145                 150                 155                 160

Pro Asp Ser Gln Glu Phe Asp Ser Glu Glu Glu Ala Val Glu Tyr Ser
                165                 170                 175

Glu Leu Glu Asp Ser Gly Ala Gly Thr Cys Ala Glu Glu Thr Val Ser
            180                 185                 190

Ser Ser Thr Asp Ala Arg Pro Cys Thr
        195                 200

<210>  10
<211>  204
<212>  PRT
<213>  homo sapiens

<400>  10

Met Asn Pro Ser Met Lys Gln Lys Gln Glu Glu Ile Lys Glu Asn Ile
1               5                   10                  15

Lys Asn Ser Ser Val Pro Ala Ala Ala Pro Ser Ala Ser Gly Ser Leu
            20                  25                  30

Val Gly Arg Glu Asn Glu Leu Ser Ala Gly Leu Ser Lys Arg Lys His
        35                  40                  45

Arg Asn Asp His Leu Thr Ser Thr Thr Ser Ser Pro Gly Val Ile Val
    50                  55                  60

Pro Glu Ser Ser Glu Asn Lys Asn Leu Gly Gly Val Thr Gln Glu Ser
65                  70                  75                  80

Phe Asp Leu Met Ile Lys Glu Asn Pro Ser Ser Gln Tyr Trp Lys Glu
                85                  90                  95

Val Ala Glu Lys Arg Arg Lys Ala Leu Tyr Glu Ala Leu Lys Glu Asn
            100                 105                 110

Glu Lys Leu His Lys Glu Ile Glu Gln Lys Asp Asn Glu Ile Ala Arg
        115                 120                 125

Leu Lys Lys Glu Asn Lys Glu Leu Ala Glu Val Ala Glu His Val Gln
    130                 135                 140

Tyr Met Ala Glu Leu Ile Glu Arg Leu Asn Gly Glu Pro Leu Asp Asn
145                 150                 155                 160

Phe Glu Ser Leu Asp Asn Gln Glu Phe Asp Ser Glu Glu Glu Thr Val
                165                 170                 175

Glu Asp Ser Leu Val Glu Asp Ser Glu Ile Gly Thr Cys Ala Glu Gly
            180                 185                 190

Thr Val Ser Ser Ser Thr Asp Ala Lys Pro Cys Ile
        195                 200

<210>  11
<211>  606
<212>  DNA
<213>  Mus musculus

<400>  11
atgaatctca gtatgaagca gaagcaggag ggagcccaag agaatgtgaa gaatagtcct      60

gtcccagcgg ccgcgccttc tgcagatgga tctcttgttg gcagagaaaa tgagttgcca     120

aaaggcttgt tcaaaaggaa gctttgggat gaccagctag catctcagac ttcaagctgt     180

ggtccagaag ctaatgaaaa taaggatgtt ggagacctca cccaggaagc ctttgatctt     240

ataagtaaag agaacccatc ttctcagtat tggaaagaag tggcagagca gcggaggaaa     300

gctctctacg aagcactgaa agagaatgag aaacttcata aagaaattga acaaaaggac     360

agtgagattg cccgcctgag aaaggagaat aaagacttgg cagaagtagc tgagcacgtg     420

cagtacatgg cggaggtaat cgagaggctg agtaatgaac ctctggataa ctttgaatca     480

ccggatagtc aggaatttga ttctgaagaa gaagctgttg agtattcaga actggaagac     540

tcaggagctg ggacgtgtgc tgaagagact gtgtcttcct ccacggatgc caggccgtgt     600

acatga                                                                606

<210>  12
<211>  690
<212>  DNA
<213>  mus musculus

<400>  12
atgaccatgg actacaagga cgacgatgac aagctcgatg gaggataccc ctacgacgtg      60

cccgactacg ccggaggact cgagatgaat ctcagtatga agcagaagca ggagggagcc     120

caagagaatg tgaagaatag tcctgtccca gcggccgcgc cttctgcaga tggatctctt     180

gttggcagag aaaatgagtt gccaaaaggc ttgttcaaaa ggaagctttg ggatgaccag     240

ctagcatctc agacttcaag ctgtggtcca gaagctaatg aaaataagga tgttggagac     300

ctcacccagg aagcctttga tcttataagt aaagagaacc catcttctca gtattggaaa     360

gaagtggcag agcagcggag gaaagctctc tacgaagcac tgaaagagaa tgagaaactt     420

cataaagaaa ttgaacaaaa ggacagtgag attgcccgcc tgagaaagga gaataaagac     480

ttggcagaag tagctgagca cgtgcagtac atggcggagg taatcgagag gctgagtaat     540

gaacctctgg ataactttga atcaccggat agtcaggaat ttgattctga agaagaagct     600

gttgagtatt cagaactgga agactcagga gctgggacgt gtgctgaaga gactgtgtct     660

tcctccacgg atgccaggcc gtgtacatga                                      690